(19) Europäisches Patentamt

European Patent Office

Office européen des brevets

(11) Publication number: **0 508 221 A1**

## (12) EUROPEAN PATENT APPLICATION

(21) Application number: **92105164.5**

(22) Date of filing: **25.03.92**

(51) Int. Cl.⁵: **C07K 15/14**, C12P 21/08,
A61K 37/02, A61K 39/00,
A61K 49/00, A61K 39/395,
C12N 15/12

(30) Priority: **28.03.91 US 677009**
**07.01.92 US 817921**

(43) Date of publication of application:
**14.10.92 Bulletin 92/42**

(84) Designated Contracting States:
**AT BE CH DE DK ES FR GB GR IT LI LU NL PT
SE**

(71) Applicant: **AMERICAN CYANAMID COMPANY
1937 West Main Street P.O. Box 60
Stamford Connecticut 06904-0060(US)**

(72) Inventor: **Eppler, Cecil Mark
162 Bateman Road
Langhorne, Pennsylvania 19047(US)**
Inventor: **Zysk, John Ronald
RD2, Box 183, Route 519
Frenchtown, New Jersey 08825(US)**
Inventor: **Corbett, Martin John
112 Union Street
Mt. Holly, New Jersey 08060(US)**
Inventor: **Shieh, Hong-Ming
35 Pine Glen Road
Langhorne, Pennsylvania 19047(US)**
Inventor: **Hadcock, John
4 Canby Court
West Islip, New York 11795(US)**
Inventor: **Strnad, Joann
1433 Wheatsheaf Road
Yardley, Pennsylvania 19067(US)**
Inventor: **Hulmes, Jeffrey David
23 Welch road
Ringwood, New Jersey 07456(US)**

(74) Representative: **Wächtershäuser, Günter, Dr.
Tal 29
W-8000 München 2(DE)**

(54) **Somatostatin receptor.**

(57) The invention relates to a purified somatostatin receptor which is purified at least about 30,000-fold over the receptor which is membrane-bound. The invention also provides pharmaceutical compositions containing the receptor and antibodies to the receptor, and isolated nucleic acid sequences encoding the receptor protein.

BACKGROUND OF THE INVENTION

All vertebrates produce one or more different types of growth factors or growth hormones which are responsible for, among other effects, stimulating protein synthesis, cell division and growth, tissue formation, repair and/or maintenance, and storage or release of particular necessary nutrients into or out of specific cells. Such factors or hormones are often proteins or polypeptides; they share the common feature of being manufactured and released by one type of cell, but exerting their ultimate effects on a different type of target cell. Among some of the better known growth mediators are nerve growth factor, epidermal growth factor, fibroblast growth factor, insulin-like growth factors (somatomedin) and growth hormone (somatotropin).

Each of these factors acts initially by binding to a receptor protein, which may be located either on the surface or in the cytoplasm of the particular factor's target cell. The receptor has a binding site which has a high affinity and specificity for the growth factor or hormone; when the binding between factor and receptor occurs, a sequence of reactions is initiated which in some manner alters the functioning of the target cell. For example, it may cause the target cell to increase production and secretion of a particular protein, or alternately, it may signal the target cell to temporarily cease or decrease production of a certain protein.

Another example of a specific type of intercellular signalling compound is the peptide somatostatin, also referred to as somatotropin release-inhibiting factor (SRIF). This peptide is produced in a number of tissues, including the brain, gut, pancreas and adrenal cortex, and has wide-ranging effects throughout the body. As its name implies, SRIF inhibits the release of somatotropin from the anterior pituitary. However, other physiological effects of SRIF include inhibition of glucagon and insulin release from the pancreas, regulation of gut motility, and neurotransmission/neuromodulation function in the brain. In the latter capacity, SRIF can stimulate the release of neurotransmitters such as serotonin, dopamine and epinephrine. SRIF exists in two principle forms in mammalian tissues: SRIF-14, which is a tetradecapeptide, and SRIF-28, in which residues 15-28 are identical to SRIF-14. SRIF-28 also has an additional 14 amino acids at its amino terminus. The shorter peptide results from posttranslational proteolytic cleavage of SRIF-28.

The intracellular aspect of the signalling action of somatostatin is mediated via receptors on the surface of target cells. It has been suggested that SRIF receptor subtypes exist in different cells and for differential interactions with specific signalling mechanisms. For example, radiological competition assays have shown that SRIF-14, SRIF-28 and other synthetic analogs can show vast differences in receptor binding affinity in brain, pancreas and pituitary cells (Srikant et al., Endocrinology 108:341-343, 1981; Tran et al., science 228:492-495, 1985; Heiman et al., Neuroendocrinology 45:429-436, 1987; Amhardt et al., J. Clin. Invest. 80:1455-1460, 1987; Srikant et al., Nature 294:259-260, 1981). SRIF receptor subtypes are also suggested by the observation of differential effects of SRIF-14 and SRIF-28 on glucagon and insulin release in pancreas (Brown et al., Endocrinology 108:2391-2393, 1981) and on K$^+$ channel activation in cultured cerebral cortical neurons (Wang et al., PNAS USA 86:9616-9620, 1989).

The activity of the SRIF receptor in mediating the SRIF biological effects seas to be intimately associated with pertussis toxin-sensitive GTP-binding regulatory proteins (hereinafter referred to as G proteins). It has been suggested (Jacobs et al., PNAS USA 80:3899-3902, 1983; Lewis et al. PNAS USA 83:9035-9039, 1988: Wang et al., PNAS USA 86:9616-9620, 1989) that SRIF receptors couple to cellular effector systems such as the adenylyl cyclase complex and to ion conductance channels by way of the G proteins. This association has been particularly well demonstrated in pituitary cells, wherein pertussis toxin blocked SRIF-mediated inhibitions of Ca$^{2+}$ influx (Lewis et al., supra, Reisine et al., J. Pharmacol. Exp. Ther. 235:551-557, 1985) and adenylate cyclase (Reisine et al., J. Pharmacol Exp. Ther. 232:275-282, 1985), reduced agonist binding affinity of the SRIF receptor (Reisine, supra), and blocked the SRIF-mediated inhibiting of adenylate cyclase and Ca$^{2+}$ and decreased binding of [$^{125}$I]Tyr$^1$-SRIF by more than 95% (Kirk et al., Endocrinol. 114:1784-1790, 1984).

There has been evidence that direct immunoneutralization of SRIF enhances growth in primitive breeds of sheep (Spencer, Domestic Animal Endocrinology 3:55-68, 1985). However, this technique has had very limited success in commercial breeds of farm animals (Mears, can. J. Anim. Sci. 70:1091-1097, 1990). This failure could be the result of compensatory overproduction of SRIF, variability in the immune response between animals or undesirable effects on other SRIF-dependent systems (i.e., lack of tissue specificity). However, the potentiation of growth hormone releasing factor effects in the rat model system by passive immunoneutralisation of SRIF (Wehrenberg et al., Endocrinology 114:1613-1616, 1984; Wehrenberg et al., Biochem. Biophys. Res. Comm. 109:562-567, 1982) suggests that neutralization of SRIF, either pharmacologically or immunologically, has great potential. One way in which the problems with the antisomatostatin approach might be overcome is the specific antagonism of the SRIF receptor via receptor-specific ligands or antireceptor antibodies. Use of either one of these techniques involves binding a non-SRIF material to the receptor, thereby blocking the receptor site and preventing the sending of endogenous SRIF. However, to date there has been little success in synthesising peptide SRIF antagonists, and a random screening procedure would be useful in identifying compounds that inhibit SRIF activity. Moreover, although the immunological approach is becoming a means of animal growth regulation, the appropriate tools for application of these methods to SRIF have not yet been developed. In each of these approaches, the availability of a purified, well-characterised, tissue-specific receptor is essential to successful use of the methods.

Notwithstanding the need in art for isolation of SRIF receptors generally, and a pituitary receptor in particular, there has been little progress made in conclusively solubilizing and purifying well-defined SRIF receptors from any tissue type. Purification of pituitary receptors in general has been difficult because of the scarcity of tissue, as well as the problems involved in solubilizing the receptors in active form and developing an efficient purification method. SRIF receptors from various cell sources have been solubilized (He et al., Mol. Pharmacol. 37:614-621, 1990; Knuhtsen et al., J. Biol. Chem. 265:1129-1133, 1990; Reye-Desmars et al., J. Biol. Chem. 264:18789-18795) in active, high affinity states in the detergent CHAPS. SRIF Receptor: [125I] SRIF complexes have also been solubilized in CHAPS (Knuhtsen et al., Biochem. J. 254:6410647, 1988) and Zwittergent (Zeggari et al., Eur. J. Biochem. 165:667-673, 1987). At best, however, these complexes were stable enough to allow chromatographic separation of free from bound radioligand, and no further purification of receptor was reported.

One attempt to purify the solubilized SRIF receptor from rat brain has been reported (He et al., PNAS USA 86:1480-1484, 1989). The authors employed affinity chromatography on immobilised D-Trp-SRIF14. Eluates from the affinity column were not shown to have SRIF binding activity. Nonetheless, these investigators concluded that the SRIF receptor had been isolated. This conclusionwas based on the ability to chemically cross-link a [125I]-labelled SRIF analog to a 55-60,000 MW protein in the eluate, and comparison with a similar sized protein which was radiolabelled by chemical cross-linking in intact brain membranes (Thermos and Reisine, Mol. Pharmacol 33:370-377, 1988). The human gut SRIF receptor from HGT-1 cells was said to be purified by a monoclonal antibody with apparent specificity for the SRIF receptor (Reye-Desmars, et al., J. Biol. Chem. 264:18789-18795, 1989). Eluates from the immunoaffinity column showed binding of [125I]SRIF, and also contained a single narrow 90,000 MW band. However, the affinity column eluates showed greatly lowered SRIF binding affinity, making characterization of the purified receptor less reliable. Also, the sharply focused 90K band was unusual for a presumably glycosylated protein. Proteins of this type usually give a poorly focused "fussy" appearance. No further information has been published on any of these putative receptors; therefore, their identity as such remains uncertain.

The present invention now provides a substantially pure SRIF receptor protein, the identity of which is verified by a number of different criteria. The receptor protein, which is purified by a novel method, is isolated substantially free of other associated proteins, including G proteins, and as such is useful for sequencing, gene cloning, antibody production, and therapeutic purposes.

## SUMMARY OF THE INVENTION

The present invention relates to a substantially pure somatostatin (SRIF) receptor protein, and biologically active fragments thereof. The receptor is purified from 30,000 to 100,000-fold relative to the membrane-bound receptor. By "biologically active fragments" is meant natural or synthetic portions of the full length receptor which are essential in conferring immunogenicity to the molecule or which are themselves capable of binding receptor-specific ligand. In a preferred embodiment, the receptor is derived from human, rat, sheep, pig or cow. Somatostatin receptors are isolatable from pituitary, brain, gut, pancreas, liver and lung. In a particularly preferred embodiment, the receptor is derivable from pituitary tissue. The pituitary receptor is a heavily glycosylated glycoprotein with a molecular weight of about 75-95,000, preferably in the range of 80-90,000 daltons, with a core protein having a molecular weight of about 35-40,000 daltons.

The invention also relates to pharmaceutical compositions comprising an effective amount of the pure receptor or fragment in combination with a pharmaceutically acceptable carrier. The receptor and receptor fragments are useful in screening methods to identify somatostatin analogs, as well as identifying compounds which may act as somatostatin antagonists at the receptor site. They also are useful in raising somatostatin-receptor specific antibodies; such anti-bodies may, by blocking the receptor site, effectively prevent somatostatin binding and thereby enhance growth. Pharmaceutical compositions containing the receptor or receptor fragments can be used to treat any disorder resulting from or associated with an excess of circulating somatostatin, such as pancreatic somatostatinoma, where diabetes mellitus, reduced growth hormone levels, and gut maladsorption result from excess circulating SRIF levels (Wass, in, Endocrinology, 2nd ed., Vol. 1:152-166, 1989; L.G. DeGroot, ed., W.B. Saunders Co.). Such compositions can be employed for in vivo administration to bind circulating somatostatin, also thereby preventing its binding to the endogenous receptor, and enhancing growth. The invention thus also relates to these therapeutic methods for regulating or modulating the action of somatostatin in vivo, by use of such pharmaceutical compositions.

The invention further relates to an isolated nucleic acid sequence encoding a somatostatin receptor and vectors and host cells useful in recombinant production of the receptor.

The SRIF receptor of the present invention is isolated by a novel purification method. This method is claimed in copending and cofiled U.S. Serial No. 07/677,003, incorporated herein by reference in its entirety.

## BRIEF DESCRIPTION OF THE FIGURES

Figure 1 illustrates the relative potencies of various synthetic biotinylated SRIF ligands in a competitive binding assay with non-biotinylated ligands. (A) shows a compression with biotinylated ligand with no spacer; (B) shows a comparison of biotinylated ligands with spacers.

Figure 2 illustrates a comparison of the relative inding affinities of three biotinylated ligands compared with a non-biotinylated ([$^{125}$I]Tyr11-S14) ligand.

Figure 3 illustrates the EDTA/EGTA/GTP-gamma-S eluate from a streptavidin column run on 12% SDS-PAGE. The diffuse band with molecular weight of between 75-95,000 daltons represents the SRIF receptor glycoprotein, while the two narrowly focused bands, molecular weights of 40,000 and 35,000, are the associated G protein subunits.

Figure 4 illustrates the results of an experiment in which samples equivalent to those shown in Figure 6 are reacted with pertussis toxin and [$^{32}$P]NADH (see text); [$^{32}$P] labelled proteins are separated on 12% SDS-PAGE and visualized by autoradiography. A protein of about 40K is only seen in the sample with Bio-S28 specifically bound ("-10$^{-5}$ S14") prior to purification of receptor on SA-A, confirming the identity is a G protein.

Figure 5 illustrates the contents of the EDTA/EGTA/GTP-gamma-S eluate after application to WGA agarose and elution with 10 mM sugar. A diffuse band showing the expected molecular weight glycoprotein is clearly shown when the WGA eluate is run on 12% SDS-PAGE.

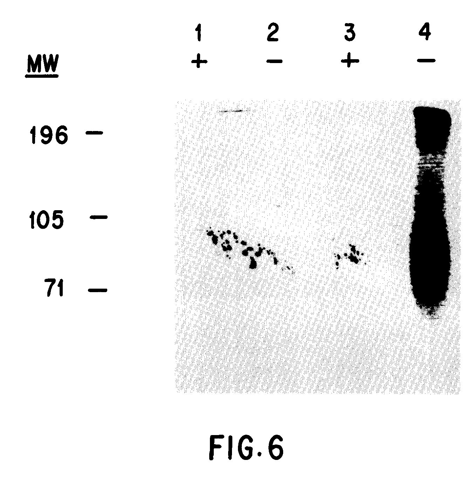

Figure 6 illustrates the chemical cross-linking of (Leu8,DTrp22,[$^{125}$I]Tyr25)S28 to the SRIF receptor and interaction of the solubilized, cross-linked R:L complex with wheat germ agglutinin. Binding of the [$^{125}$I] labelled S28 analog to GB$_4$C$_1$ membranes and chemical cross-linking of the radiolabelled R:L complex with ANB-NOS are as described in "Methods". Binding incubations are done in the presence (" + ") or absence ("-") of 10$^{-6}$ M cold S14. The membranes re then solubilized in solubilization buffer + 0.15% deoxycholate:lysolecithin + protease inhibitors. Binding of solubilized material to WGA-agarose and elution with N-N'-N''-triacetylchitotriose ("TAC") are as described in the text. Prior to SDS-PAGE on 12% gels, the samples are concentrated on Centricon-30 filters and extracted in CHCl$_3$:MeOH:H$_2$O as described in "Methods". Exposure time is 14 days. The numbered samples are shown in pairs + or - 10$^{-6}$ cold S14 in binding incubations and are as follows: 1.2 = Non-bound to WGA-agarose, 3,4 = Eluted from WGA-agarose by 4 mM TAC.

Figure 7 illustrates deglycosylation of SRIF receptor:[$^{125}$I]S28 cross-linked complexes with endoF. A. = deglycosylation of radiolabelled cross-linked R:L complex in membranes. "Control", lane 1 = no enzyme; lanes 2-5 = 0.2, 0.5, 1 and 2.5 ul of endoF. Visualization of labelled proteins by autoradiography. B. The R:L complex is first separated by SDS-PAGE, electroeluted, and the deglycosylated before final electrophoresis.

Figure 8 depicts a partial nucleic acid and amino acid sequence of the pituitary somatostatin receptor. This represents a portion of the receptor spanning intracellular loop and transmembrane domain VII (see Table 4 for further details).

Figure 9 depicts full length nucleic acid and amino acid sequence of a pituitary somatostatin receptor.

Figure 10 depicts a portion of the nucleic acid and amino acid sequence of a brain somatostatin receptor predicted to have about 392 amino acids in the full length protein. The ↓ in the sequence indicates a gap in the sequence presented.

Figure 11 depicts a western blot of Endo-F digested SRIF receptor probed with rabbit anti-LNETTETQR sera. Purified SRIF receptor is digested 18 hours at 37°C with 0.05 units of Endoglycosidase-F, run on a 12% SDS-PAGE gel, and transferred to PVDF membrane. The blot Is stained after transfer for total protein with colloidal gold (a). Lanes 1 and 4, mol. wt. standards: Lanes 2 and 5, 0.05 units of Endo-F; Lanes 3 and 6, SRIF receptor after Endo-F digestion. Endo-F has a mol. wt. of 32 kDa as has previously been reported.

Most of the purified SRIF receptor has been deglycosylated to its lower mol. wt. form of 38 kDa. Duplicate membranes are probed with rabbit preimmune (Lanes 1, 2, 3) and immune (Lanes 4, 5, 6) at a 1:50 dilution and detected with $^{125}$I-donkey antirabbit and autoradiography (b). Both preimmune and immune sera reacted nonspecifically with the Endo-F enzyme at 32 kDa: however, the rabbit anti-LNETTETQR sera specifically recognizes the deglycosylated SRIF receptor at 38 kDa in Lane 6 as indicated (←).

## DETAILED DESCRIPTION OF THE INVENTION

As described in the subsequent Examples, SRIF receptor is isolatable in adequate quantity from rat GH$_4$C$_1$ cells, a pituitary cell line. However, the source of the receptor is not limited to these cells but in fact may be derived from any cell line or tissue expressing the receptor. Potentially useful pituitary cell lines would include the GH$_3$ rat pituitary tumor line (Yasumura et al., science 154:1186, 1986) and the AtT-20 mouse pituitary tumor line (Sabol, Arch. Biochem. Biophys. 203:37-48, 1980). Other useful cell lines would be the HGT-1 human gastric carcinoma line (Laboisse et al., Cancer Research 42:1541-1548, 1982) and the rat pancreatic acinar cell line AR4-2J (Viguerie et al., Am. J. Physiol. 255:G113-G120, 1988). Useful tissues include intact pituitary, liver, and brain as well as isolated pancreatic acini from a number of species. The receptor is preferably derived from human, porcine, bovine, ovine and murine tissues or cell lines.

The receptor is isolated initially as a complex with its associated G protein. Although a variety of SRIF analogs are available and could be used as ligands for receptor binding, it is preferable that biotinylated SRIF analogs are prepared to be used as ligands for purification. A particularly preferred ligand is the analog referred to as BioS28 or Bio-SRIF28, which is biotinyl-NH-Leu8-DTrp22-Tyr25-SRIF28 (Peninsula Labs). In the preferred isolation method, the ligand is first bound to intact pituitary cell membranes to form a receptor: ligand (R:L) complex. After binding, the membranes are solubilized in detergent and intact receptor:ligand complexes are obtained. A particularly useful detergent for this purpose is a combination of deoxycholate and lysolecithin, preferably in a ratio of 1:1, at a concentration of 0.2% W/V or less. This assumes a membrane protein concentration of 1 mg/ml. At this stage, the receptor portion of the complex consists of the receptor and its associated G protein consisting of alpha, beta, and gamma subunits; this is confirmed by the rapid dissociation of the R:L complex in the presence of chelating agents EDTA and EGTA and a stable GTP analog (GTP-gamma-S). The recovery of soluble intact R:L complex is generally in the range of 40-70% of that initially present in the membranes after the binding step.

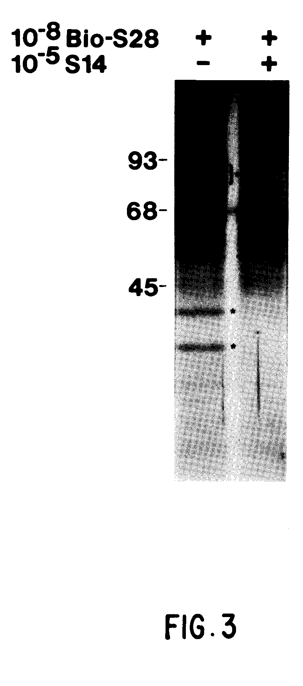

The solubilized R:L complex is then contacted with streptavidin-agarose (SA-A), whereby the biotinylated portion of the R:L complex will tightly bind to the streptavidin. Streptavidin is preferred, due to its lower non-specific binding: however, free and immobilized avidin is also available (Pierce, Vector) and may be suitable for some purposes. The SA-A is eluted with EDTA, EGTA and GTP-gamma-S. GTP-gamma-S serves to dissociate the G protein from its the receptor, thereby lowering the affinity of the receptor and indirectly causing dissociation from the ligand. EDTA and EGTA may add to this effect and also directly interfere with ligand binding, which depends on divalent cations. The eluate (in which the receptor is purified to a level of at least about 25-30%), when run on 12% SDS-PAGE, shows a diffuse band, the glycoprotein receptor, with a molecular weight of about 75,000-95,000 daltons, and two narrow bands, having molecular weight of about 40,000 and 35,000 daltons, representing two of the dissociated G protein subunits, G$_i$- or G$_o$-alpha and G-beta, respectively (Figure 3). The presence of the G protein subunits provides further evidence of the identity of the larger protein as the SRIF receptor. The SRIF receptor band and the two G protein subunits do not appear in eluates from control SA-A columns done with SRIF receptors loaded with non-biotinylated S14. Electroelution at this stage yields intact glycoprotein at a purity of about 80-90%. Although this level of purification may be adequate for analytical purposes such as amino acid sequencing, in an alternate embodiment, it is desirable to further purify the receptor by lectin affinity chromatography rather than gel electrophoresis. This is a high efficiency, high purification step which yields a receptor of 90% or greater purity (as judged by SDS-PAGE). The protein core of the receptor can be obtained from this preparation by removal of carbohydrate by the ensyme endoF and purification of the core receptor by SDS-PAGE or reverse phase HPLC. This final purification of the protein core yields a 35-40,000 MW core receptor.

Additional experiments, utilizing non-biotinylated radiolabelled SRIF analog ligands, are conducted to independently confirm true receptor isolation and to further characterize the chemical nature of the receptor. A [$^{125}$I]Tyr11-S14 analog is used as described above, to create soluble R:L complexes from GH$_4$C1 membranes. These complexes are applied to wheat germ agglutinin (WGA) and eluted with N-N'-N''-triacetylchitotriose. About 82% of the original soluble R:L complex is recovered in the eluate, confirming that the isolated material is expected to be a glycoprotein (Table 3).

In a second experiment, a (Leu-8,Trp-22[$^{125}$I]Tyr25)S28 analog ([$^{125}$I S28]) is chemically cross-linked to the receptor, solubilized, and the complex then adsorbed to WGA-agarose and eluted as noted above. On SDS-PAGE, the eluate shows the radiolabelled receptor as a broad band having a molecular weight of about 80-100,000 daltons (Figure 6), with about 3,000 daltons being due to the presence of the ligand.

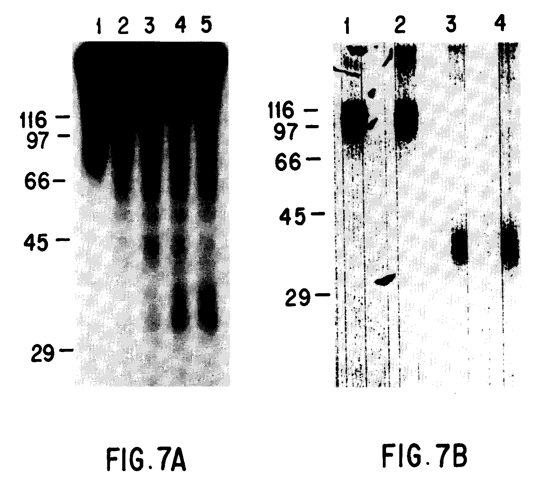

To obtain a more accurate molecular weight of the protein core of SRIF receptor, the cross-linking of [$^{125}$I S28] with the receptor is followed by deglycosylation with the enzyme endoF (a 1:1 mixture of endoglycosidase F and N-glycosidase F in either one of two ways. First, deglycosylation is carried out on SDS-solubilized membranes before electrophoresis. The final product has a molecular weight of about 38,000 daltons (Figure 7), in contrast to the diffuse band described above. Alternately, the radiolabelled R:L complex is first separated by gel electrophoresis, removed from the gel by electroelution, and then deglycosylated before final separation by gel electrophoresis. The result is essentially the same as with the crude membrane proteins, although the final product forms a broader band of 35-40,000 daltons, possibly due to incomplete deglycosylation or from chemical heterogeneity caused by processing.

Thus, based on data from the putative purified receptor and the radiolabelled cross-linked SRIF analogs, the pituitary SRIF receptor is confirmed as a glycoprotein with a molecular weight of about 75-95,000. MW of the glycosylated receptor inherently falls in a broad range due to microheterogeneity of the attached oligosaccharide groups. Approximately 50% of the apparent molecular weight of the protein is due to carbohydrate, with the core receptor protein having a molecular weight of about 35,000-40,000 daltons.

The purified receptor, or biologically active fragments thereof, can be used for a number of purposes. For example, the purified material, in either glycosylated or unglycosylated form, can be used to create monoclonal or polyclonal antibodies having specificity for the SRIF receptor. The technology for creation of monoclonal antibodies is well known in the art (see, e.g., Goding, Monoclonal Antibodies: Principles and Practice, 2nd Ed., 1986). Such antibodies may have utility in tumor localization and therapy: it has recently been reported that SRIF analogs have proven useful in imaging certain receptor-bearing endocrine tumors (Lemberts et al., N. Engl. J. Med., November 1, 1990, 1246-1249). It is reasonable to anticipate that detectably-labelled antireceptor antibodies can serve the same purpose, by targeting SRIF receptor sites. They can also be used in tumor therapy, preferably in conjugated form with antitumor or cytotoxic drugs or radioisotopes, which by their association with the antibody, will be targeted directly to cancerous cells expressing large amounts of receptor.

Anti-receptor antibodies can also be administered in a passive immunization protocol, to bind receptor, thus blocking SRIF from binding to receptor sites. This ultimately should enhance growth of the host so treated. To a similar end, the receptor can be used in the preparation of anti-idiotypic antibodies. Such antibodies would bind to circulating SRIF, thus preventing its binding to receptor sites.

Pharmaceutical compositions containing the receptor and/or active fragments thereof, in combination with a pharmaceutically acceptable carrier, may also be prepared. In one embodiment, such compositions will contain an amount of receptor and/or fragment effective to bind circulating SRIF, thereby preventing it from interacting with endogenous receptors in vivo. In a second embodiment, the composition contains an immunogenic amount of receptor or fragment; such compositions, when administered in vivo to an animal, may be used to raise anti-receptor autoantibodies that will bind to endogenous receptors, thereby preventing circulating SRIF from binding the receptors. Such immunological methods are disclosed in U.S. Patent No. 4,857,637, relating specifically to the use of growth hormone (somatotropin) receptors; the contents of this patent are incorporated herein by reference. The effect in both of these cases is to inhibit the effects of SRIF, thereby leading to increased release of somatotropin, and ultimately increased growth of the animal so treated. Other effects of SRIF may similarly be altered, such as its effect on release of neurotransmitters and gut motility. Administration of the compounds is preferably parenteral, i.e., intramuscular, intravenous and intraperitoneal or subcutaneous; choice of carrier can be made in accordance with the preferred mode of administration, and formulation and dosage are within the ability of the skilled artisan.

The isolated receptor protein itself can be used in screening assays to identify compounds that act as analogs. For example, the receptor protein can be immobilized by any means which does not interfere with SRIF binding activity. The immobilized receptor is then contacted with a specific compound or mixture and its ability to compete with radiolabelled SRIF for binding to the receptor is evaluated. Variations on this method will be apparent to those skilled in the art.

The present invention encompasses the SRIF receptor protein and its biologically active fragments produced by any means, whether synthetically, recombinantly, or by purification of the native protein. The isolated SRIF receptor, as described above, is pure enough to be used in protein sequencing procedures which are well known in the art, and such sequencing is routinely accomplished using such methods. The protein sequence in turn is used to design oligonucleotide probes which are used to screen λgt10 libraries containing the relevant cDNA (copies of RNA), e.g., from $GH_4C_1$ pituitary cells. Hybridization of oligos with the library identifies the clone(s) containing the SRIF receptor gene or portions thereof. The gene or gene fragments are isolated from the clones, the whole gene reconstructed and then ligated into an appropriate vector by known methods. Suitable expression vectors are selected based upon the choice of host cell. Numerous vectors suitable for use in transforming bacterial cells are well known. For example, plasmids and bacteriophages such as λ phage, are the most commonly used vectors for bacterial hosts, and for E. coli in particular. In both mammalian and insect cells, plasmid and virus vectors are frequently used to obtain expression of exogenous DNA. In particular, mammalian cells are commonly transformed with SV40 or polyoma virus or with a pRC/CMV plasmid; and insect cells in culture may be transformed with baculovirus expression vectors. Yeast vector systems include yeast centromere plasmids, yeast episomal plasmids and yeast integrating plasmids. The invention encompasses any and all host cells transformed by the claimed genes, as well as expression vectors used to achieve this transformation. Also it is contemplated that transgenic animals comprising mutated receptors or antisense mRNA to alter receptor function in vivo can be created.

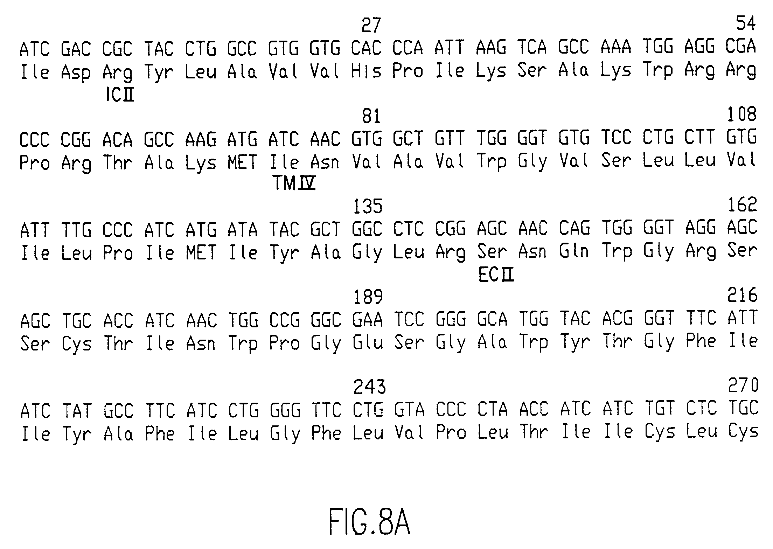

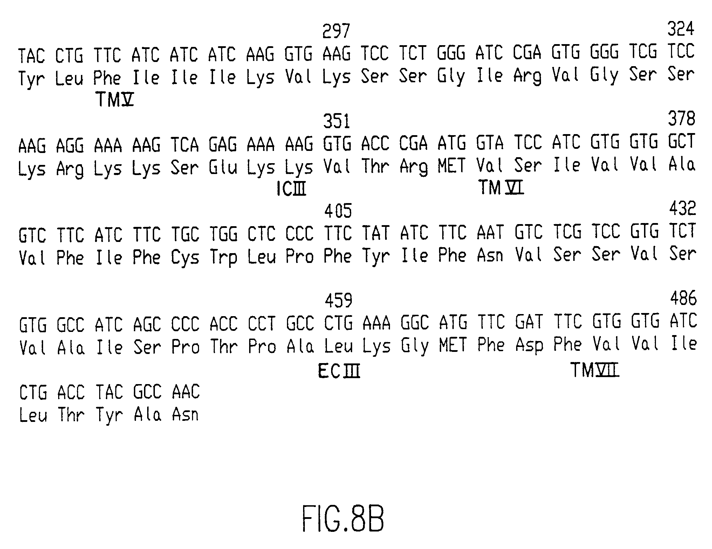

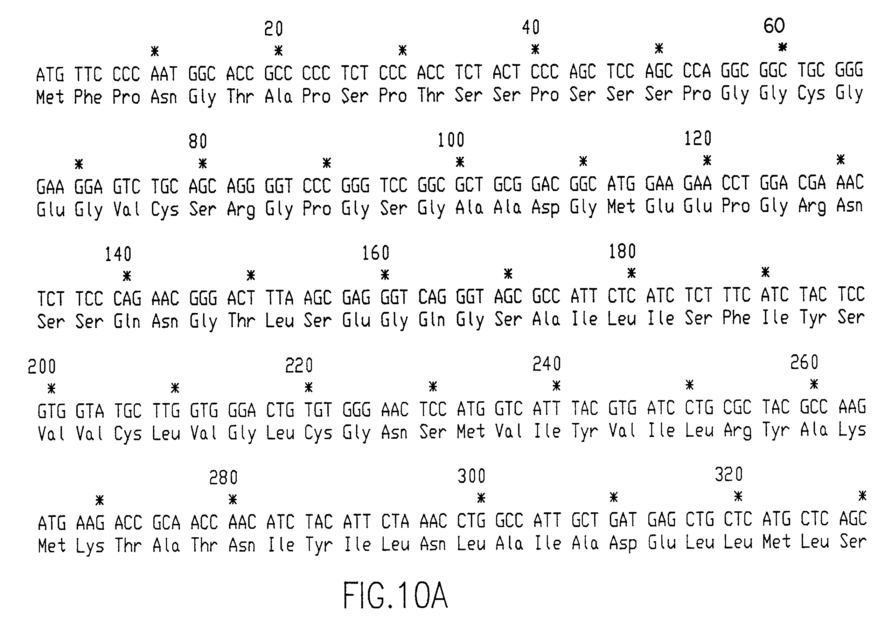

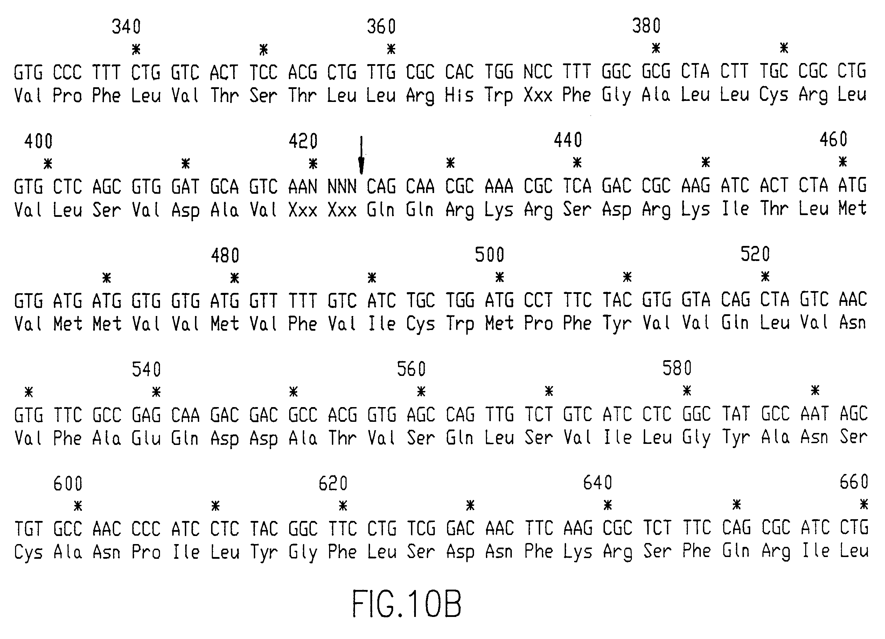

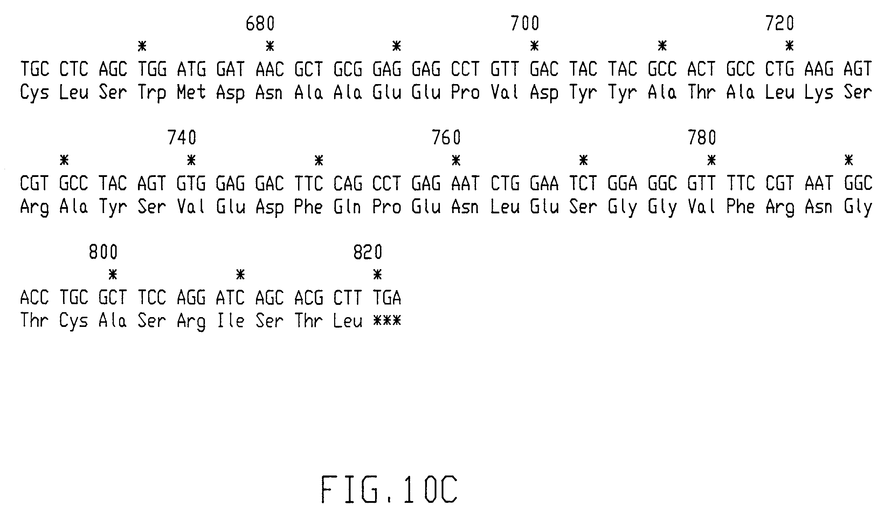

Exemplary nucleotide and amino acid sequences of the various encoded proteins have been provided in Figures 8, 9 and 10. However, it will be understood that the invention is not limited solely to those sequences, but also encompasses biologically equivalent sequences. Modifications to the sequence, such as deletions, insertions, or substitutions in the sequence which produce silent changes in the resulting protein molecule are also contemplated. For example, alteration in the gene sequence which reflect the degeneracy of the genetic code, or which result in the production of a chemically equivalent amino acid at a given site, are contemplated; thus, a codon for the amino acid alanine, a hydrophobic amino acid, may be substituted, by a codon encoding another less hydrophobid residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a biologically equivalent product. Nucleotide changes which result in alteration of the N-terminal and C-terminal portions of the protein molecule would also not be expected to alter the activity of the protein. It may also be desirable to eliminate one or more of the cysteines present in the sequence, as the presence of cysteines may result in the undesirable formation of multimers when the protein is produced recombinantly, thereby complicating the purification and crystallization processes. Each of the proposed modifications is well within the routine skill in the art, as is determination of retention of biological activity of the encoded products. Therefore, where the phrases "somatostatin receptor nucleotide sequence or gene", or "somatostatin receptor protein" are used in either the specification or claims, each will be understood to encompass all such modifications and variations which result in the production of a biologically equivalent protein. In particular, the invention contemplates those DNA sequences which are sufficiently duplicative of the sequences depicted in Figures 8, 9 and 10 so as to permit hybridization therewith under standard Southern hybridization conditions, such as those described in Maniatis et al. (Molecular Cloning. A Laboratory Manual. Cold Spring Harbor Laboratory, 1989), as well as the biologically active proteins produced thereby. It will readily be recognized that the nucleic acid sequences of the specific Figures can readily be employed in Southern hybridization procedures to identify and isolate somatostatin receptors in cells from a variety of species and tissue types. Such host cells and their derivatives can be used in recombinant production of all or a portion of the somatostatin receptor.

The following non-limiting examples further illustrate the present invention.

## EXAMPLES

## I. METHODS

Unless otherwise stated, the following are used in the examples provided below:

**A. Synthesis of Peptides** - Four biotinylated SRIF analogs are synthesised. Two analogs, biotinyl[NH-$(CH_2)_5$-CO]-NH-(Tyr11)SRIF14 ( = "Bio-6C-S14") and biotinyl-[NH-$(CH_2)_5$-CO]$_2$-NH-(Tyr11)SRIF14 ( = "Bio-12C-S14") are synthesised at Applied Biosystems, Foster City, CA. Bio-6C-S14 is synthesised on solid phase by the FMOC method. Bio-12C-S14 are synthesized on solid phase by the TBOC method. The aminocaproate spacers are added by use of BOC-aminocaproate with DCC-HOBT coupling. The N-terminal biotin is also coupled by DCC-HOBT. Biotinyl-NH-(Leu8, D-Trp22, Tyr25)SRIF28 is synthesized at Peninsula Labs, Belmont, CA, on solid phase by the t-BOC method. Biotin is coupled to the N-terminal by DCC-HOBT. Biotinyl-NH-SRIF14 is synthesized at American Cyanamid, Agricultural Research Division, Princeton, NJ. This is done on solid phase by the t-BOC method. Biotin is coupled to the N-terminus by DCC-HOBT.

**B. Pituitary Cell Culture** - $GH_4C1$ rat pituitary tumor cells are initially grown as monolayers in 82.5% Dulbecco's MEM (Gibco) + 15% heat inactivated horse serum + 2.5% heat inactivated fetal bovine serum (sera from CC Labs, Cleveland, OH) + 50 units/ml penicillin + 50 ug/ml streptomycin. The cells are then placed in suspension culture by replacement of MEM by suspension culture medium (MEM modified for suspension culture of "S-MEM", Gibco) and culturing in spinner flasks (Bellco, Vineland, NJ). The concentration of horse serum is gradually reduced to 11% and the medium is supplemented with HEPES buffer and extra glucose. The medium finally developed for optimal growth of the $GH_4Cl$ cells in suspension culture is as follows (expressed in % of total volume per liquid component): 84.5% DMEM + 11% horse serum + 2.5% fetal bovine serum + 1% HEPES buffer (pB 7.4; 10 mM final conc.) + 1% penicillin/streptomycin solution (5,000 units/ml pen + 50 ug/ml strep) + 0.7% 45% glucose solution. Cells are grown at 37° C in the presence of 6% $CO_2$. Cultures are initially seeded at 1.5-2 x $10^5$ cells/ml and grown to concentrations of 6-$10^5$ cells/ml (3-4 days growth). Cells are passed by dilution or complete medium change every 3-4 days. Viability is greater than 95% by trypan blue staining.

**C. Membrane Isolation** - 1-8 liter batches of cells at densities of 6-10 x $10^5$ cells/ml are pelleted in conical bottom glass centrifuge bottles (600 or 800 ml; Bellco) at 1,000 x g for 5 min. The supernatants are carefully poured off and the cells are resuspended in ice cold homogenization medium (1 mM Na-bicarbonate at pB 7.6, 1 mM EDTA and 1 mM EGTA) containing 0.7% (vol/vol) of the "100X 4pase" protease inhibitor cocktail (see below). Twenty ml of homogenization medium is used for every liter of suspension culture. After 5 min. on ice, the hypotonically swollen cells are homogenized with 10 strokes of a tight fitting Dounce homogenizer (Kontes, type A pestle). The homogenate is centrifuged at 1,000 x g for 10 min. and the supernatant is removed and kept on ice. The 1,000 x g pellet containing residual intact cells, nuclei and DNA is washed by gently homogenizing with 4 strokes with a type B pestle in one half the original volume of homogenization medium and recentrifuging for 10 min. at 1,000 x g. The final 1,000 x g pellet consists mostly of DNA and is discarded. The 1,000 x g supernatants are combined and centrifuged at 20,000 x g for 30 min. The 20,000 x g pellet is washed twice in 25 a Tris (pH 7.4), with centrifugation for 25 min. at 20,000 2 g. Final membrane pellets are resuspended in 25 mM Tris buffer to concentrations of 4-10 mg membrane protein/ml. Then the 100x 4pase protease inhibitor cocktails are added to 1% of final volume and aliquots are frozen on dry ice. Membranes are stored at -90°C until needed. Membrane protein is assayed with the Bradford dye binding assay (Bio-Rad).

**D. Protease Inhibitor Mixtures** - Three different protease inhibitor mixtures were used for receptor binding assays and receptor purification. A. 40X PMSF (phenylmethyl sulfonyl fluoride)/Baci = 2 mg/ml PMSF (Bachem) + 2 mg/ml bacitracin (Sigma) dissolved in DMSO. B. 400 X PMSF/Bacitracin = 20 mg/ml PMSF + 20 mg/ml bacitracin. Mixtures A and B are used in routine binding assays and in the binding step of the receptor purification protocol. The 40X concentration is generally used for smaller binding assays where pipetting accuracy of smaller volumes is a factor. Final DMSO concentrations in the binding assays, 0.25-2.5% do not affect ligand binding or any subsequent procedures. C. 100X 4Pase - 7.5 mg/ml leupeptin (Bachem) + 14.5 mg/ml PMSF = 3 mg/ml chymostatin (Bachem) + 1 mg/ml pepstatin A (Sigma) dissolved in DMSO. This mixture is used in membrane solubilization buffers and in all buffers used in receptor purification. All protease inhibitor mixtures are stored as frozen aliquots at 4-10° C and are added to buffers at appropriate dilutions immediately before use.

**E. ReceptorBinding Methods**

1. Standard Binding Assays - Binding assays are done in a binding buffer containing 50 mM HEPES (pH 7.4), 0.5% BSA and 5 mM $MgCl_2$. The standard assay for [$^{125}$I]SRIF analog binding to $GH_4C_1$ membranes, done in 96 well microtiter plates (Dynataech Immulon II Removawell plates), is carried out as follows: 1. Radioligand is diluted in binding buffer + PMSF/Baci to the desired cpm per vol. of 50 $\mu$l and then 50 $\mu$l aliquots are added to the wells. For non-specific binding samples, 5 $\mu$l of 40 $\mu$M cold S14 is also added per well. 2. Binding is initiated by adding 150 $\mu$l per well of membrane diluted to the desired concentration (10-30 ug membrane protein/well) in binding buffer + PMSF/Baci. Plates are then covered with Linbro mylar plate sealers (Flow Labs) and placed on a Dynatech Microshaker II and binding is allowed to proceed at room temperature for 1-2 hours. Binding is stopped by centrifuging the plate for 15 minutes at 2,000 x g. The supernatants are dumped off and membrane pellets washed once by addition of 200 $\mu$l of ice cold binding buffer, brief shaking and recentrifugation. Finally the individual wells are placed in 12x75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used:

2. Competitive radioligand binding assays with a concentration range of cold ligand vs. [$^{125}$I]SRIF are carried out as described above with one modification. All dilutions of SRIF analogs being assayed are made in 40XPMSF/Baci to a concentration 40X the final concentration in the assay. This gives very consistent results with a wide variety of SRIF structural analogs over a wide range of dilutions. 5 ul samples of peptide are then added per microtiter well. Membranes and radioligand are diluted out in binding buffer without protease inhibitors. Radioligand is added and mixed with cold peptides and then binding is initiated by addition of membranes.

3. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.

4. Larger scale binding assays to obtain membrane pellets for studies on solubilization of receptor:ligand complex and for receptor purification are also carried out. These are identical to the standard assays except that: (a) Binding is carried out in polypropylene tubes in volumes from 1-250 ml, (b) Concentration of membrane protein is always 0.5 mg/ml, (c) For receptor purification, BSA concentration in the binding buffer is reduced to 0.25% and the wash step is done with binding buffer without BSA. This is to reduce BSA contamination of the purified receptor.

**F. Chemical Cross-Linking of Radioligand to Receptor** - After a radioligand binding step as described above ("Receptor Binding Methods, 3."), the membrane pellets are resuspended in 200 ul per microtiter plate well of ice-cold binding buffer without BSA. Then 5 ul per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, CA) at a distance of 5-10 cm. Then the samples are transferred to Eppendorf microfuge tubes, the membranes pelleted by centrifugation, supernatants removed and membranes solubilized in Laemmli SDS sample buffer for polyacrylamide gel electrophoresis (PAGE). PAGE is done as described below and radiolabelled proteins are visualized by autoradiography of the dried gels with Kodak XAR film and Dupont image intensifier screens.

**G. Membrane Solubilization** - Initial solubilization studies are carried out in buffer containing 25 mM Tris (pH 8), 10% glycerol (wt. vol.) and 0.2 mM $CaCl_2$. Later, $MgCl_2$ is substituted for $CaCl_2$, as mentioned in the text. This will be referred to as "solubilization buffer". The highly soluble detergents including Triton X-100, deoxycholate, deoxycholate:lysolecithin, CHAPS and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer + 100,000 x g for 30 minutes. The supernatants are removed and held on ice and the pellets are discarded.

**H. Assay of Solubilized Receptors** - After binding of [$^{125}$I]SRIF analogs and solubilization of the membranes with detergent, the intact R:L complex could be assayed by four different methods (all carried out on ice or in a cold room at 4-10° C): (1) Column chromatography (Knuhtsen et al., Biochem. J. 254:641-647, 1988). Sephadex G-50 columns (8x250 mm) are equilibrated with solubilization buffer containing detergent at the concentration used to solubilize membranes and 1 mg/ml bovine serum albumin. 0.2-0.5 ml samples of solubilized membranes are applied to the columns and eluted at a flow rate of about 0.7 ml/minute. 0.18 ml samples are collected. Radioactivity is determined in a gamma counter as described under "Receptor Binding Methods". Void volumes of the columns are determined by the elution volume of blue dextran. Radioactivity eluting in the void volume (>50,000 MW) was considered bound to protein. Radioactivity eluting later, at the same volume as free [$^{125}$I]SRIF, is considered non-bound. (2) Polyethyleneglycol precipitation (Cuatrecasas, PNAS USA 69:318-322, 1972) For a 100 ul sample of solubilized membranes in a 12x75 mm polypropylene tube, 0.5 ml of 1% (w/v) bovine gamma globulin (Sigma) in 0.1 M sodium phosphate buffer is added, followed by 0.5 ml of 25% (w/v) polyethyleneglycol (Sigma) and mixing. The mixture is held on ice for 15 minutes. Then 3 ml of 0.1 M sodium phosphate (pH 7.4) is added per sample and the samples are rapidly (1-3 seconds) filtered over Whatman GF/B glass fiber filters and washed with 4 ml of the phosphate buffer. PEG precipitated SRIF receptor:[$^{125}$I]SRIF complex is determined by gamma counting of the filters. (3) GFB/PEI filter binding (Bruns et al., Analytical Biochem. 132:74-81, 1983). Whatman GF/B glass fiber filters are soaked in 0.3% polyethyleneimine (PEI, Sigma) for 3 hours. 25-100 ul samples of solubilized membranes are replaced in 12x75 mm polypropylene tubes. Then 4 ml of solubilization buffer (without detergent) is added per sample and the samples are immediately filtered through the GFB/PEI filters (1-3 seconds) and washed with 4 ml of solubilization buffer. CPM of SRIF receptor:[$^{125}$I]SRIF complex adsorbed to filters are determined by gamma counting. (4) Charcoal/Dextran (Paul and Said, Peptides 7[Suppl. 1]: 147-149, 1986). 0.5 gm of Dextran T70 (Pharmacia) is dissolved in 1 liter of water and then 5 gm of activated charcoal (Norit A, alkaline; Fisher scientific) is added. The suspension is stirred for 10 minutes at room temperature and then stored at 4° C until use. To measure R:L complex, 4 parts by volume of charcoal/dextran suspension are added to 1 part by volume of solubilized membrane. The samples are mixed and held on ice for 2 minutes and then centrifuged for 2 minutes at 11,000 x g in a Beckman microfuge. Free radioligand is adsorbed charcoal/dextran and is discarded with the pellet. SRIF receptor:[$^{125}$I]SRIF complex remain in the supernatant and is determined by gamma counting.

## I. Receptor Purification

**1. Binding of biotinyl-SRIF to GH$_4$C1 membranes.** The binding step is carried out as described in section 4 of "Receptor Binding Methods". Incubations are for 1 hour at room temperature. In the standard purification protocol, the binding incubations contain 10 nM Bio-S29/ [$^{125}$I]Bio-S28 is added as a tracer at levels of 5,000-100,000 cpm per mg of membrane protein. Control incubations contain 10 uM cold S14 to saturate the receptor with non-biotinylated ligand.

**2. Solubilization of receptor:ligand complex.** This is done as described ("Membrane Solubilization"), with 0.15% deoxycholate:lysolecithin in solubilization buffer containing 0.2 mM MgCl$_2$, to obtain 100,000 x g supernatants containing solubilized R:L complex.

**3. Adsorption of solubilized R:L complex to streptavidin.** Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10° C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer + 0.15% deoxycholate:lysolecithin + 1/500 (vol/vol) 100x4pase.

**4. Elution of streptavidin column.** The column is eluted with solubilization buffer + 0.1 mM EDTA + 0.1 mM EGTA + 0.1 mM GTP-gamma-S (Sigma) + 0.15% (wt/vol) deoxycholate:lysolecithin + 1/1000 (vol/vol) 100x4pase. First, one column volume of elution buffer is passed through the column and flow is stopped for 20-30 minutes. Then 3-4 more column volumes of elution buffer are passed through. All the eluates are pooled.

**5. Wheat germ agglutinin purification of receptor** - Eluates from the streptavidin column are incubated overnight (12-15 hours) with immobilized wheat germ agglutinin (WGA agarose, Vector Labs) to adsorb the SRIF receptor via interaction of covalently bound carbohydrate with the WGA lectin. The ratio (vol/vol) of WGA-agarose to streptavidin column eluate is generally 1:400. A range from 1:1000 to 1:200 gave very similar results. After the binding step, the resin is pelleted by centrifugation, the supernatant is removed and saved, and the resin is washed 3 times (about 2 minutes each) in buffer containing 50 mM HEPES (pH 8), 5 mM MgCl$_2$ and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10mM N-N'-N''-triacetylchitotriose in the same HEPES buffer (vide supra) used to wash the resin. After each elution step, the resin is centrifuged down and the supernatant is carefully removed, free of WGA-agarose pellets. The three, pooled eluates contain the final, purified SRIF receptor. The material non-bound to WGA contain G protein subunits specifically eluted from the streptavidin column plus non-specific contaminants. All these fractions are stored frozen at -90° C.

## J. Miscellaneous Preparative and Analytical Methods

**1. SDS-polyacrylamide gel electrophoresis.** Electrophoretic separation of proteins, solubilized in 1% SDS (in Laemmli sample buffer) + 5 mM dithiothreitol for 5-10 minutes at 90°C, is done in 12% SDS-polyacrylamide gels by the method of Laemmli (Nature 227:680-685, 1970). Stacking gels are composed of 3.8% polyacrylamide. For regular silver staining of proteins bands the gels are fixed in 40% methanol + 10% acetic acid and then stained with the Bio-Rad silver staining kit (Bio-Rad Labs). For silver staining of glycoproteins, the gels are stained by the method of Jay et al. (Analytical Biochem. 185:324-330, 1990), with prestaining by the dye alcian blue. This method is necessary for silver staining of heavily glycosylated proteins such as the SRIF receptor.

**2. Concentration and extraction of protein samples for analysis.** Prior to gel electrophoresis, amino acid analysis and sequencing, samples are concentrated in Centricon-30 microconcentrators (Amicon Co.). One to two ml samples are placed in the microconcentrator tubes and centrifuged at 3,000 x g to pass excess buffer through the filters. Samples are concentrated to volumes of 50-150 ul and transferred to 1.5 ml, Eppendorf microfuge tubes. Then the samples are extracted in $CHCl_3$:MeOH:$H_2O$ to remove detergents and buffer and obtain a dry protein pellet (Wessel and Flugge, Analytical Biochem. 138:141-143, 1984). This pellet could be solubilized in SDS sample buffer for PAGE or in other solvents such as 70% formic acid or 8M urea for other purposes such as generation of proteolytic peptides, amino acid analysis and sequencing.

**3. Preparation of radioligands for receptor binding assays.** SRIF analogs are radioiodinated by the chloramine-T method. The reagents are added to 1.5 ml siliconized Eppendorf centrifuge tubes as follows: (a) 5 ul of peptide (0.5 mg/ml) in 50 mM potassium phosphate buffer (pH 7.4), (b) 5 ul of 100 mM potassium phosphate buffer (pH 7.4), (c) 5 ul of methanol, (d) 4 ul of Na[$^{125}$I] (Amersham, 100 uDi/ml; cat. = IMS.30), mix by vortexing, add reagent (e) 5 ul of 0.7 mM chloramine-T (Kodak), mix by vortexing and allow 20 seconds reaction time, add (f) 5 ul of 2 mM tyrosine in 0.1% TFA. Immediately after reaction, the samples are injected onto a Supelco LC-308 column (c-8 reverse phase, 5 u particle size, 300 angstrom pore size, column dimensions = 0.46 x 5 cm). Labelled peptides are eluted isocratically at 20-26% acetonitrile (depending on the peptide) in water/0.1% TFA. Monoiodinated SRIF analogs are very efficiently separated by noniodinated peptide by this method. We have established this by kinetic studies with nonradioactive iodide and correlation with radiolabelling patterns. Therefore, the monoiodinated analogs are considered to have specific radioactivities of 2,200 Ci/mmole, the same as [$^{125}$I]. 0.1 ml radioactive peptide fractions off the column are collected into 0.1 ml volumes of 2% BSA in 1% acetic acid. The most active fractions are pooled, aliquoted and stored frozen at -20% C.

## II. RECEPTOR ISOLATION USING BIOTINYLATED LIGANDS

### Receptor-Binding Characteristics of Biotinyl-SRIF Analogs

Four biotinylated SRIF analogs are synthesized ("Methods: Synthesis of Peptides"). Their structures and abbreviated designations are as follows:

Biotinyl-NH-SRIF14 = Bio-S14

Biotinyl-[NH-$(CH_2)_5$-CO]-NH-(Tyr11)SRIF14 = Bio-6C-S14

Biotinyl-[NH-$(CH_2)_5$-CO]$_2$-NH-(Tyr11)SRIF14 = Bio-12C-S14

Biotinyl-NH-(Leu8, D-Trp22, Tyr25)SRIF28 = Bio-S28

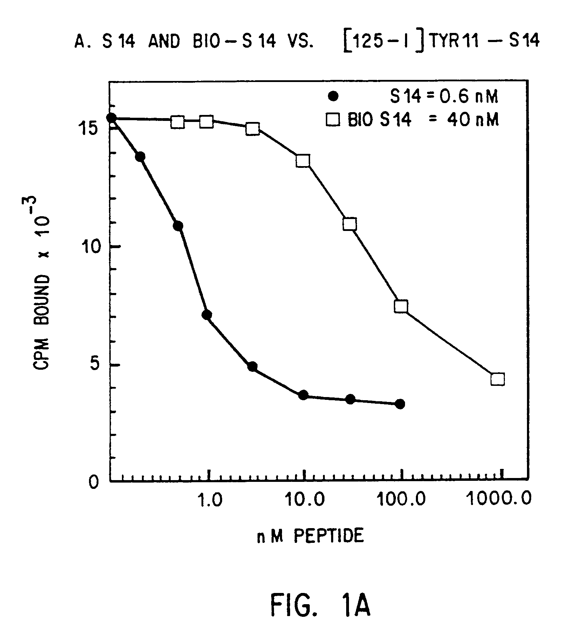

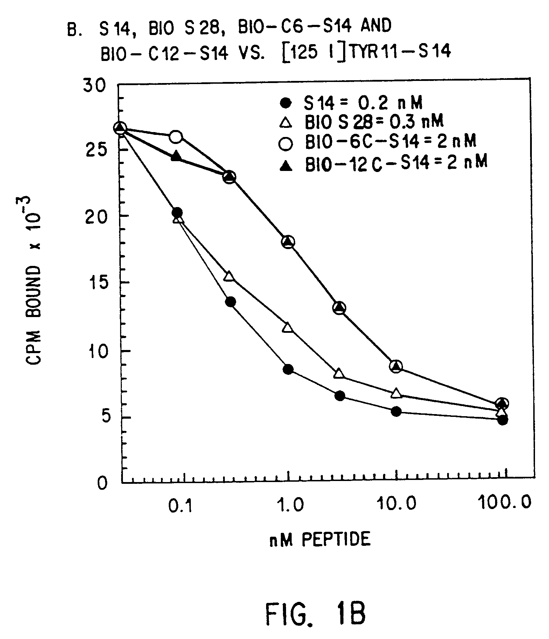

Figure 1 shows the potencies of these peptides relative to each other and to S14 in competitive binding assays with [$^{125}$I]Tyr11-S14 and GH$_4$C$_1$ membranes. These assays are carried out as described in Methods: Receptor Binding Methods, 2". The first SRIF analog synthesized, Biotinyl-NH-S14, contains no spacer between the biotinyl and S14 moieties and has only about 1.5% the potency of S14 in the competitive binding assay (Figure 1A; assays contained to 40 ug of GH$_4$C$_1$ membrane protein and 85,000 cpm of radioligand). Therefore the Bio-6C-S14, Bio-12C-S14 and Bio-S28 analogs are preferably synthesized. In Bio-S28, amino acid residues 1-14 are considered a spacer since residues 15-28 are equivalent to an S14 analog, having all the necessary structure for high affinity binding. The three spacer-containing biotinyl SRIFs show receptor binding activity similar to that of S14 (Figure 1B; assays contained 20 ug of GH$_4$C$_1$ membrane protein and 100,000 cpm of radioligand). The IC$_{50}$s and relative potencies are S14 (0.2 nM) > Bio-S28 (0.3 nM) > Bio-6C-S14 (2 nM) >Bio-12C-S14 (2 nM).

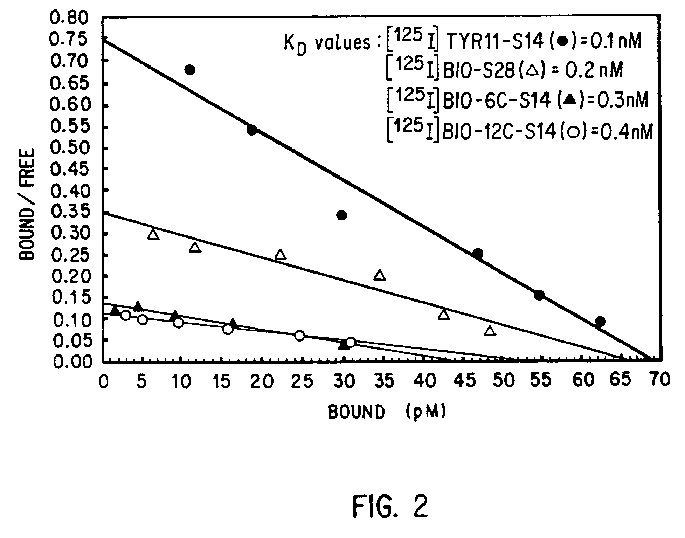

Because Bio-C6-S14, Bio-C12-S14 and Bio-S28 all contain tyrosine residues, it is possible to prepare their [$^{125}$I] labelled analogs and carry out Scatchard analyses of their binding affinities relative to [$^{125}$I]Tyr11-S14 (Figure 2). As shown in the figure, the [$^{125}$I]-labelled peptides fall in the same order of potency as their non-labelled forms. The dissociation constants (KDs) and relative binding affinities are [$^{125}$I]Tyr11-S14 (0.1 nM) > [$^{125}$I]Bio-S28 (0.2 nM) > [$^{125}$I]Bio-6C-S14 (0.3 nM) > [$^{125}$I]Bio-12C-S14 (0.4 nM).

### Streptavidin Binding Characteristics of Biotinyl-SRIF Analogs

The three, high-affinity binding, biotinyl-SRIF analogs (Bio-6C-S14, Bio-12C-S14 and Bio-S28) all appear to be useful for SRIF receptor purification. However, Bio-S28 is selected for further use in SRIF receptor purification because it binds to the SRIF receptor with the highest affinity (Figures 1 and 2) and because the solubilized R:L complex made with [$^{125}$I]Bio-S28 binds somewhat better to immobilized streptavidin than the R:L complexes with Bio-6C-S14 and Bio-12C-S14 (see Table 1).

To compare the three biotinyl-SRIF analogs in terms of binding to streptavidin, the following experiment is done. [$^{125}$I]Bio-SRIF analogs are bound to GH$_4$C$_1$ membranes as described ("Methods: Receptor Binding Methods, 3"). All radioligands are at a concentration of 0.77 to $10^6$ cpm/ml. For each radioligand, a control for non-specific binding was done in the presence of 1 uM cold S14. CPM bound/mg membrane protein after the binding step are as follows: [$^{125}$I]Bio-6C-S14 = 755,020 (total) and 43,361 (non-specific); [$^{125}$I]Bio-12C = S14 = 633,134 (total) and 39,538 (nonspecific); [$^{125}$I]Bio-S28 = 1,065,512 (total) and 35,049 (nonspecific). The membrane pellets are solubilized in solubilization buffer containing 0.15% deoxycholate:lysolecithin and 0.2 mM MgCl$_2$ as described ("Methods: Membrane Solubilization"). It should be noted that Mg$^2$ can replace Ca$^2$+ in the solubilization buffer, giving equally effective recovery of intact R:L complex and reducing the possibility of Ca$^{2+}$-dependent proteolysis. One ml samples of solubilized membranes are incubated with 0.05 ml vols of streptavidin-agarose at 4-10° C on a tube rotator for the times shown. Non-specific binding samples are not done due to the low levels of non-specific binding. At the times shown, the SA beads are spun down and 100 ul samples of supernatant are counted. The cpm are compared to initial cpm in the sample and % binding to SA is calculated from this. The results are presented in Table 1. Also, it should be noted that binding of cpm from the supernatant is considered to parallel the binding of R:L complex. Several observations confirm that this is a valid assumption.

## Table 1

### Binding of [$^{125}$I]-Labelled Biotinyl-SRIFs to Immobilized Streptavidin

CPM are determined for 100 ul samples of initial 100,000 x g supernatant (containing soluble R:L complex) and supernatant after incubation with SA-agarose for the times shown.

| | | % Binding of Radioligand to SA-Agarose = (CPM Non-bound / Initial CPM) | | | |
| | | 1 hour | | 3 hours | |
| Radioligand | Initial CPM | CPM | % Bound* | CPM | % Bound |
|---|---|---|---|---|---|
| [$^{125}$I]Bio-6C-S14 | 63,490 | 28,381 | 55% | 16,228 | 74% |
| [$^{125}$I]Bio-12C-S14 | 54,344 | 23,391 | 57% | 13,071 | 76% |
| [$^{125}$I]Bio-S28 | 92,534 | 31,645 | 66% | 17,098 | 82% |

* Calculated as ... 100% - [(Non-bound CPM / Initial CPM) x 100%]

Purification of SRIF Receptor

A preparation of SRIF receptor is purified from 17 mg GH$_4$C$_1$ pituitary cell membranes as described in "Methods: Receptor Purification". Some important features of this experiment are as follows: Two 17 mg samples of membranes are used. Both are incubated with 10$^{-8}$ M Bio-S28. However, one sample also receives 10$^{-5}$ M non-biotinyl S14 for 2-3 minutes before addition of any Bio-S28. This serves to block binding of Bio-S28 and creates a control to show non-specific binding of proteins to the streptavidin column. Also, each sample receives 1.5 x 10$^8$ cpm of [$^{125}$I]Bio-S28 as a tracer. This is added 2-3 minutes before the addition of cold Bio-S28. After a 1-hour binding step, the membranes are washed in binding buffer without BSA and solubilized in solubilization buffer containing 0.15% deoxycholate:lysolecithin and 0.2 mM MgCl2. Each 100,000 x g supernatant (17 ml) is incubated with 0.6 ml of streptavidin-agarose for 4 hours at 4-10° C. The SA-agarose is transferred to a 0.7 cm diameter column, washed and eluted with EDTA/EGTA/GTP-gamma-S as described ("Methods: Receptor Purification).

Radioligand is followed through the procedure to estimate solubilization of R:L complex and % initial binding of R:L complex to immobilized streptavidin. This is shown in Table 2 below.

TABLE 2

| Use of [125I]Bio-S28 to Trace SRIF R:L Complex During Purification on Immobilized Streptavidin | | | |
|---|---|---|---|
| Ligand in Binding Step | CPM Initially Bound to Membranes | CPM Solubilized by Deoxycholate: Lysolecithin | CPM Solubilized Non-Bound to Streptavidin |
| $10^{-8}$ M Bio-S28 | 59,132 | 54,094 (91%) | 14,960 (28%) |
| $10^{-8}$ M Bio-S28 + $10^{-5}$ M S14 (NSB) | 9,316 | 6,834 (73%) | 1,054 (15%) |

A calculation from Table 2 shows that 72% of the specifically bound radioligand is bound to streptavidin. As discussed above, this should approximate the binding of R:L complex to streptavidin.

EDTA/EGTA/GTP-gamma-S is used to elute SRIF receptor from streptavidin columns because the soluble R:L complex is dissociated by this combination of agents. For the SRIF R:L complex solubilized in 0.15% deoxycholate:lysolecithin, 0.1 mM EDTA + 0.1 mM EGTA + 0.1 mM GTP-gamma-S gives 75-90% dissociation. This is probably due to initial dissociation of G-protein from the receptor and consequent lowering of ligand binding affinity.

The EDTA/EGTA/GTP-gamma-S eluates from streptavidin are concentrated by Centricon-30 filters and solvent extraction and then solubilized in SDS and separated on 12% SDS-polyacrylamide gels ("Methods: Miscellaneous preparative and Analytical Methods, 1, 2"). Staining of the gels by alcian blue/silver ("Methods: ibid") reveals three protein bands that are specifically bound to and eluted from streptavidin (Figure 3). One is a diffuse, 75-95,000MW band. The two other specific bands are more sharply focused and had MWs of about 40,000 and 35,000. There are several non-specific bands that also appear in the sample where specific binding of Bio-S28 is blocked by a 1000-fold excess of non-biotinylated S14 (" + $10^{-5}$ S14" in Figure 3).

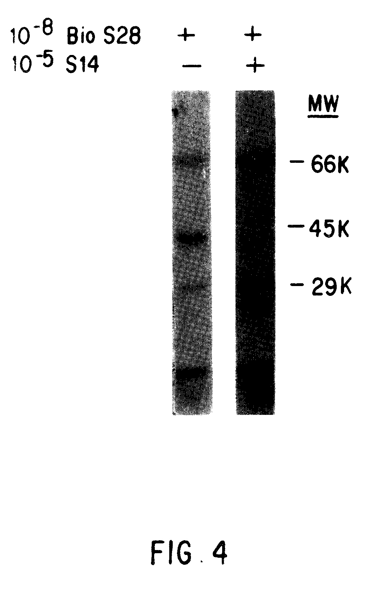

The 40K and 35K proteins have appropriate sizes for G protein alpha and beta subunits respectively. The 40K protein is functionally identified as a G-alpha subunit by the technique of ADP-ribosylation (Figure 4). Here, pertussis toxin catalyzes transfer of [23P]ADP-ribose from NADE to protein. This reaction is shown to be highly specific for G-alpha subunits of the "i" and "o" subtypes (Stadel and Lefkowitz, ibid). In Figure 4, [32P] labelling of a 40K protein in the presence of pertussis toxin and [32P]NADH occurs in the streptavidin eluates with samples initially incubated with $10^{-8}$ M Bio-S28 but not if Bio-S28 binding is blocked by excess non-biotinylated S14.

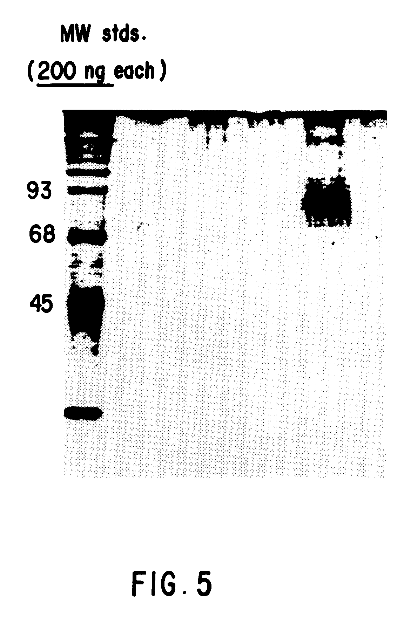

Finally, if the EDTA/EGTA/GTP-gamma-S eluate from streptavidin is incubated with immobilized wheat germ agglutinin (WGA-agarose), the putative, 75-95K SRIF receptor binds and can be eluted in nearly pure form by 10 mM N-N'-N''-triacetychitotriose (Figure 5; WGA binding and elution done as in "Methods: Receptor Purification. 5"; SDS-PAGE and alcian blue/silver staining done as previously described). Figure 5 shows 1% of a purified sample obtained from 750 mg of $CH_4C_1$ membrane protein. We can consistently obtain SRIF receptor of this purity by the methods described herein.

## III. OTHER MEANS OF RECEPTOR CHARACTERIZATION COMPARISONS WITH PURIFIED RECEPTOR

Confirmatory experiments are conducted to verify isolation of the same receptor by traditional binding methods and to further verify the chemical nature of the receptor. As the results show, a glycoprotein of identical molecular weight is identified by these methods as well. The ligands used in the following experiments are [125I]Tyr11-S14 and (Leu8, D-Trp22, [125I]Tyr25)S28, hereinafter referred to as "radiolabelled S28").

Lectin Affinity Binding

A. Solubilized Receptor: [125I]Tyr11-S14 Complex - Binding of [125I]Tyr11-Sir to $GH_4C_1$ membranes is carried out as in "Receptor Binding Methods. 4". Binding incubations contain 0.5 mg/ml membrane protein and $6 \times 10^6$ cpm/ml of [125I]Tyr11-S14. Solubilization of membranes in 0.15% deoxycholate:lysolecithin is also as previous described. Incubations for binding to WGA-agarose included 5 vol. of solubilized membrane (100,000 x g supernatant) + 1 vol. of WGA-agarose. Binding proceeds for 2 hours at 4° C with constant agitation. After binding to WGA, the supernatants are removed and replaced with equal volumes of solubilization buffers, 0.15% deoxycholate:lysolecithin, protease inhibitors, and 4 mM N-N'-N'' - triacetylchitotriose. Elution is carried out for 1 hour and the supernatants are removed. Intact R:L complex in these and other solubilized samples are assayed by the GFB/PEI filter method (Methods: Assay of Solubilized Receptors, 3"). The results are shown in Table 3, indicating the glycoprotein nature of the bound material. In this experiment, 82% of the initial solubilized R:L complex is recovered in the eluate from the WGA resin. The ratios of total bound to non-specifically found ligand (A/B) show that WGA specifically selects for the SRIF receptor. Thus, the ratio of total to non-specific binding increases about 4-fold from intact membranes to the eluate from WGA agarose. The same trend occurs with the R:L complex as measured by adsorption to GFB/PEI filters.

**TABLE 3**

| | CPM of $[^{125}I]Tyr11-S14$ | | |
|---|---|---|---|
| **Sample** | **A. Total**[1] | **B. Non-specific**[2] | **A/B** |
| **Membranes** | 151,848 | 28,812 | 5.3 |
| **100,000 x g supernatant** | 104,420 | 12,756 | 8.2 |
| **Non-bound to WGA-agarose** | 23,112 | 9,624 | 2.4 |
| **Bound to WGA-agarose** | 69,889 | 3,157 | 22.1 |
| **Eluted from WGA-agarose** | 58,925 | 2,524 | 23.0 |

**Recovery of Soluble R:L Complex:**
**CPM of Soluble $[^{125}I]Tyr11-S14$**
**Bound to GFB/PEI Filters**

| **A. Total** | **B. Non-specific** | **A/B** |
|---|---|---|
| -- | -- | -- |
| 45,307 | 4,005 | 11.3 |
| 5,787 | 1,998 | 2.9 |
| -- | -- | -- |
| 34,829 | 1,032 | 33.7 |

B. Chemically Cross-linked Receptor: Radiolabelled S28 Complex - The binding phase is carried out as noted above under "Receptor Binding Methods: 3". Chemical cross-linking is carried out as described in "Methods: Chemical Cross-linking of Radioligand to Receptor". Binding incubations are done in the presence or absence of $10^{-6}$ M cold S14. The membranes are then solubilized in solubilization buffer as defined above, with 0.15% deoxycholate:lysolecithin and protease inhibitors. Binding of solubilized material to WGA-Agarose is achieved by incubating 5 volumes of solubilized membrane (100 x g supernatant) with one volume of WGA- agarose. Binding proceeds for 2 hours at 4°C with constant agitation. After binding to WGA, the supernatants are removed and replaced with equal volumes of solubilization buffer, 0.15% deoxycholate:lysolecithin, protease inhibitors, and 4 mM N-N'-N''-triacetylchitotriose. Elution is carried out for 1 hour and the supernatants removed.

Prior to SUB-PAGE on 12% gels, the samples are concentrated on Centricon-30 filters, extracted in CHCl₃:MeOH:H₂O. These methods were carried out as described in "Methods: Miscellaneous Preparative and Analytical Procedures 1 and 2". The radiolabelled proteins on the dried gels are visualized by autoradiography at -70°C with DuPont intensifying screens. Exposure time is 14 days. Figure 6 shows the radiolabelled SRIF receptor as a broad band with a molecular weight of 75-95,000 daltons, as observed using biotinylated ligands. The specificity of this band is shown by the complete lack of radiolabelling in membranes incubated with [Leu8, D-Trp22 [$^{125}$I]Tyr25)S28 and $10^{-6}$ M cold S14 (+ lanes, 1 and 3). The glycoprotein nature of the receptor is clearly shown by the fact that it is deleted from the 100,000 x g supernatants after exposure to WGA-agarose (lane 2) and was eluted from the WGA resin by N-N'-N''-triacetylchitotriose (lane 4).

For a more accurate molecular weight determination of the SRIF receptor, the cross-linking of radioligand to the receptor is followed by deglycosylation with the enzyme endoF (a 1:1 mixture of endoglycosidase F and N-glycosidase F (Boehringer). This is carried out in two different ways. Figure 7A shows the patterns obtained when deglycosylation is carried out on SDS-solubilized membranes prior to electrophoresis. The final product is a relatively tight hand with a molecular weight of about 38,000. This contrasts sharply with the large, relatively diffuse band of intact receptor (Figure 7A). In Figure 7B, the radiolabelled receptor:ligand complex is first separated by gel electrophoresis, then removed from the gel by electroelution and then deglycosylated before final gel electrophoresis. This result is essentially the same as with the crude membrane proteins, but the final product formed a broader band with a molecular weight of about 35-40,000, possibly due to incomplete deglycosylation or chemical heterogeneity caused by processing.

## IV. ISOLATION OF SOMATOSTATIN RECEPTORS FROM OTHER TISSUE SOURCES

A recent report has indicated the possible role of glycosylation in the affinity of agonist binding to somatostatin (SRIF) receptors (J. Biol. Chem. 266:20094). We have investigated the carbohydrate composition of SRIF receptors in membranes from the rat pituitary tumor cell lines $GH_4C_1$ and $GH_3$ and porcine brain are investigated. SRIF receptors from $GH_4C_1$, $GH_3$ and porcine brain are specifically cross-linked with $[^{125}I](Leu^8, D-Trp^{22}, Tyr^{25})$SRIF28 via ANB-NOS and their carbohydrate composition evaluated by digestion with exo- and endoglycosidases. Cross-linking was competitively inhibited by increasing concentrations of cold SRIF-14 or the presence of GTP$\gamma$s. The approximate molecular weight of the cross-linked species are: $GH_4C_1$ = 90 kDa, $GH_3$ = 70 and 80 kDa, and porcine brain = 70 kDa. Solubilized cross-linked receptors are collected by binding to WGA and after elution are treated with exo- and endoglycosidases. Endo-F or N-Glycanase digestion reveals a 38 kDa core receptor present in all membrane types. The isoelectric point of the intact cross-linked receptor is 6.4 and becomes only slightly more basic after Endo-F digestion. Neuraminidase treatment of $GH_4C_1$ receptor reveals no apparent shift in molecular weight, however, there is a decrease in radiolabel signal, possibly indicating a partial cross-linking of radioligand to terminal sialic acids. Conversely, neuraminidase treatment of porcine brain receptor shows a shift in molecular weight from 70 kDa to 55 kDa. Endo-H (high mannose specific) and 0-Glycanase (0-linkage specific) digestions have no effect on the SRIF receptors. This is the first characterization of a porcine brain SRIF receptor and also the first evidence of a common deglycosylated core SRIF receptor of 38 kDa among diverse sources.

## V. AMINO ACID SEQUENCE FROM PURIFIED SRIF RECEPTOR

For this purpose, SRIF receptor is purified by affinity chromatography on streptavidin/WGA as described above. Pure receptor of the quality shown in described above is prepared from about 2 gm of $GH_4C_1$ pituitary call membrane protein. This material is concentrated to a volume of 0.15 ml on a Centricon-30 centrifugal concentrator, as previously described, and extracted by the Wessel and Flugge method in $CHC_{13}$: $MeOH:H_2O$ to remove detergent and obtain a dry protein pellet. The receptor is subjected to cyanogen bromide digestion overnight at 20°C in 0.1 ml of 70% formic acid containing 1% cyanogen bromide (both reagents obtained from Aldrich Chemical Co.). After digestion is complete, solvents and unused reagent are removed by Savant Speed-Vac. The dry pellet is washed once in water and then solubilized in 100 ul of Laemmli SDS sample buffer and the peptides are separated on a 16% SDS-polyacrylamide gel (pre-cast gel purchased from Novex, San Diego, CA) at a constant voltage of 90 volts. For this purpose, the 0.1 ml sample is run in 3 separate lanes as 0.033 ml aliquots. After electrophoresis, the gel is transferred electrophoretically onto a PVDF membrane ("Pro-Blot" membranes, Applied Biosystems Inc.). The transfer buffer contains 10 mM CAPS, pH 10, and 20% methanol and transfer is for 3.5 hours at 350 milliamps. After transfer the peptides are made visible on the membrane by staining with Coomassie brilliant Blue (Sigma). Six peptide bands with apparent MWs between 2,000 and 8,000 are excised from the membrane and analyzed for NH12 terminal amino acid sequence with an Applied Biosystems automated protein sequencer. Two of these peptides yield especially clear amino acid sequence (Figure 8 and Table 4).

Based on homology to other G protein-coupled receptors, these two fragments correspond to intracellular loop II and transmembrane domain VII (see Table 4); the sequences Asp-Arg-Tyr from peptide 1 and Asn-Pro-X-X-Tyr [X = hydrophobic] from peptide 2 are especially conserved among G protein-coupled receptors). However, search of the data bank (SwissProtein) reveals the overall sequences to be unique.

## TABLE 4

### A. AMINO ACID SEQUENCES OBTAINED

Peptide 1 =   Phe-Asp-Phe-Val-Val-Ile-Leu-Thr-Tyr-Ala-
Asn-Ser(?)-X-Ala-Asn-Pro-Ile-Leu-Tyr-
Ala-Phe-Leu(?)-X-Asp-Asn

Peptide 2 =   Ser(?)-Ile-Asp-Arg-Tyr-Leu-Ala-Val-Val-
His-Pro-Ile-Lys-Ser-Ala-Lys(?)

? = ambiguous call
X = unknown

**Note:**
Yields per cycle of amino acid residue ranges from 5 to
48 pmoles (except for serine, which was in 1-5 range).
These yields are within the range where identification
is relatively unambiguous.

### B. COMPARATIVE AMINO ACID SEQUENCES FROM OTHER G PROTEIN-COUPLED RECEPTORS

From 7th Transmembrane Domain...

| | |
|---|---|
| Dopamine1 | Asn-Pro-Ile-Ile-Tyr |
| Dopamine2 | Asn-Pro-Ile-Ile-Tyr |
| Beta1 adrenergic | Asn-Pro-Leu-Ile-Tyr |
| Beta2 adrenergic | Asn-Pro-Ile-Ile-Tyr |
| Alpha1 adrenergic | Asn-Pro-Ile-Ile-Tyr |
| Alpha2a adrenergic | Asn-Pro-Val-Ile-Tyr |
| Alpha2b adrenergic | Asn-Pro-Val-Ile-Tyr |

**Note:**
All the above have Asp-Arg-Tyr in intracellular loop II

## C. OLIGONUCLEOTIDE PRIMERS

### ICII (Sense)

**5′ ATC GAC CGC TAC CTG GCC GTG GTG CAC CC (ACT) AT (CT) AA 3′**

### TVIII (Antisense)

**5′ GTT GGC GTA GGT CAG GAT CAC CAC GAA (AG) TC (AG) AA 3′**

## VI. MOLECULAR CLONING OF SRIF RECEPTOR

A partial DNA clone of the somatostatin (SRIF) receptor has been obtained. Oligonucleotide primers are synthesised from portions of two different deduced amino acid sequences of the purified receptor and used in PCR amplification of the somatostatin receptor DNA. These primers (Table 4) are synthesized based on codon usage and a degenerate 3' end for anchoring the 3' end of each primer to template nucleic acid. PCR amplification of the partial $GH_4C_1$ somatostatin receptor DNA is performed using a Perkin Elmer Cetus DNA Thermal Cycler with a kit obtained from the company. Rat genomic DNA (100 ng), $GH_4C_1$ RNA(1 $\mu$g) $GH_4C_1$ cDNA (100 ng) in 100 $\mu$l samples are subjected to 35 cycles at 39°C for 1 minute (annealing), 72°C for 1 minute (extension), and 94°C for 50 seconds (denaturation). Following the 35 cycle PCR amplification 10 $\mu$l of each sample is amplified again for 35 cycles under the same conditions except for the annealing temperature which is increased to 50°C for 1 minute. From the deduced location of the corresponding peptides (i.e., TM VII and IC II), amplification of a 500-700 base pair fragment of the receptor cDNA is to be expected and in fact a 501 base pair fragment is obtained (Figure 8). Several lines of evidence confirm that this fragment encodes a portion of the protein purified above. First, the amino acid sequence deduced from the DNA sequence of this fragment contains significant homology to other G-protein-linked receptors, especially to a brain orphan receptor (60%) and to the neuromedin receptor (50%). Second, the amino acid sequence (167 residues) deduced from this DNA fragment contains many of the hallmarks of a G-protein-linked receptor, including membrane spanning domains IV, V, and VI and part of VII. Finally, the deduced sequence contains internal peptides that are identical to those obtained from peptide sequencing of the somatostatin receptor (SerAlaLysTrpArgArg and SerAsnGlnTrpGlyArg).

A plasmid containing the 501 base pair fragment (pJ2P) has been deposited with the American Type Culture Collection, 12301 Parklawn Drive, Rockville, Maryland 20852 USA, on January 7, 1992 as Accession No. ATCC 68887 (501 bp).

Full-length coding regions of the $GH_4C_1$ and brain somatostatin receptor cDNAs are isolated from rat genomic DNA by PCR amplifications (35 cycles) under the following conditions: annealing, 60°C, 2 minutes; extension, 72⁹C, 2 minutes; denaturation 94°C, 50 seconds. The porcine pituitary somatostatin receptor (homolog to $GH_4C_1$ receptor) is isolated using identical conditions.

The partial somatostatin receptor PCR DNA fragment is blunt ended with T4 DNA polymerase and cloned into the Eco RV site of pBluescript SK (Stratagene). The full-length somatostatin receptor cDNAs are cloned into the HindIII and XbaI sites of pBluescript SK vector for sequence analysis. Sequencing is performed on an Applied Biosystems automated DNA sequencer using dye primers.

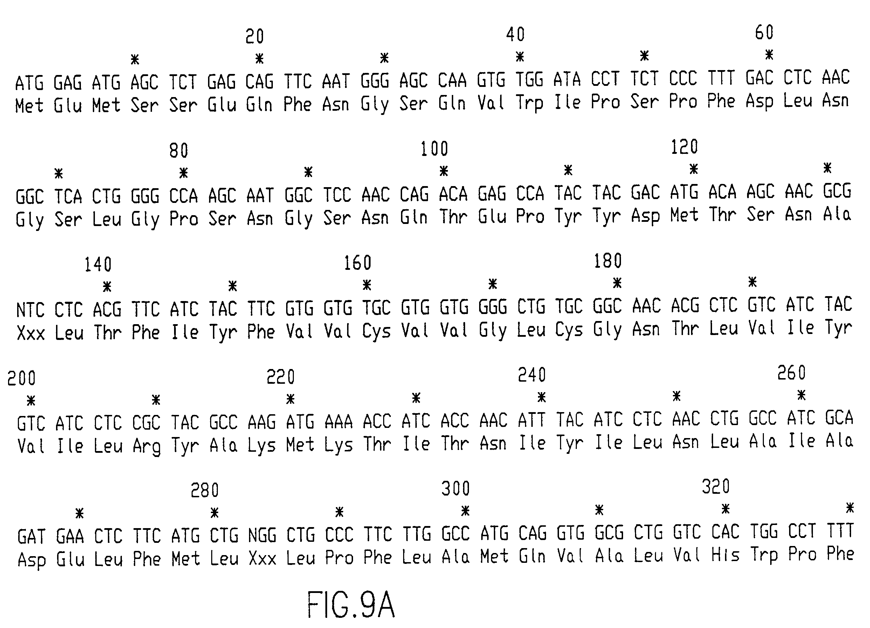

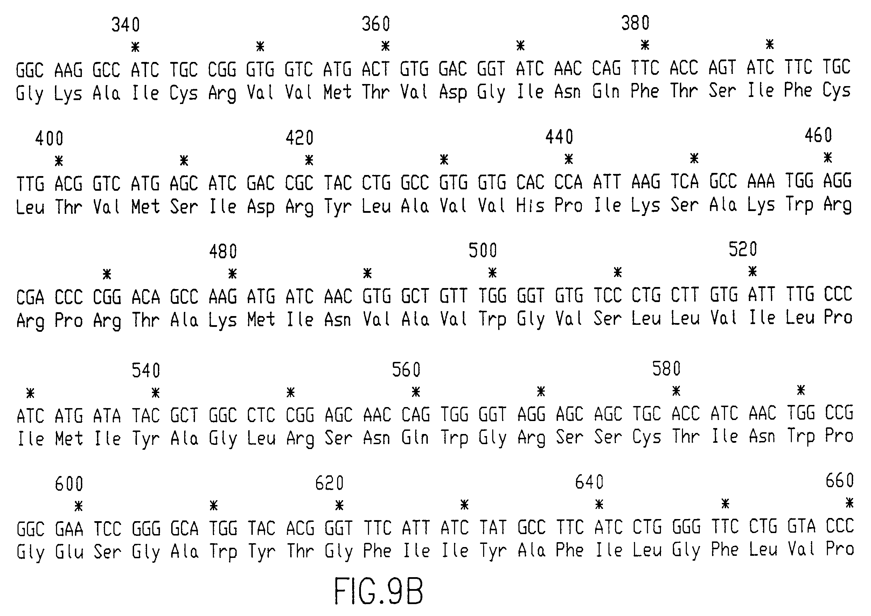

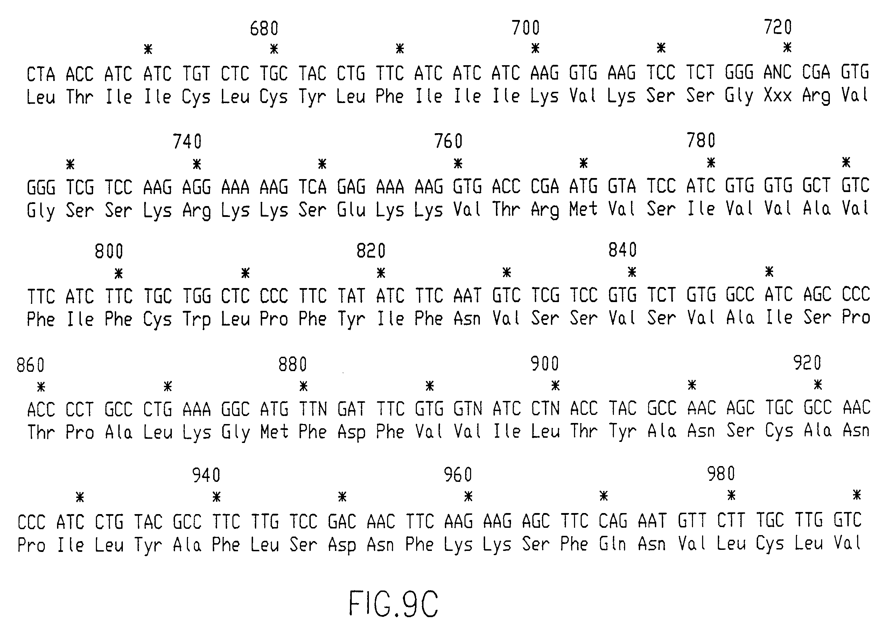

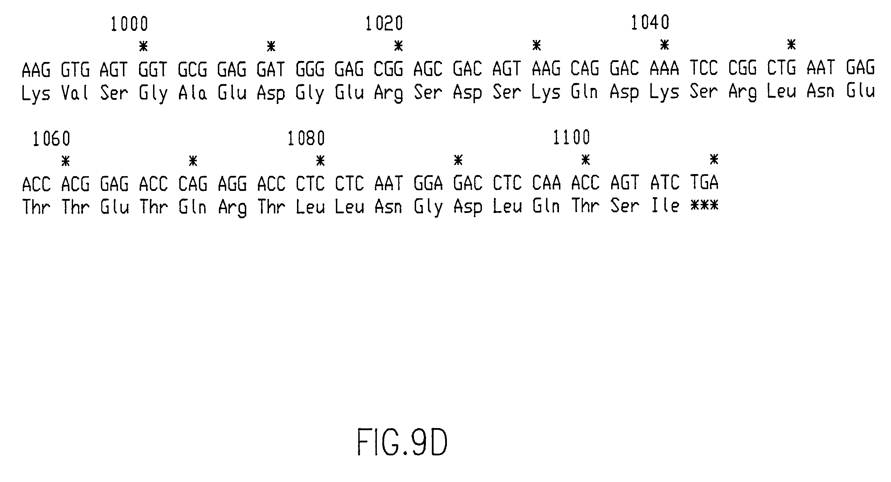

The coding region of the rat pituitary receptor contains all the amino acid sequences obtained from the sequencing of the purified $GH_4C_1$ somatostatin receptor. The full-length $GH_4C_1$ somatostatin receptor cDNA is subcloned into pBluescript for sequencing and into pRC/CMV vector for expression and pharmacological evaluation in transfected CHO cells. All other amino acid sequences obtained from the sequencing of the pituitary receptor are detected in the full-length clone (Figure 9).

The porcine homolog of the pituitary receptor is also cloned. The porcine clone contains the full coding region with the exception of the first 15 amino acids. Based on mutagenesis studies of other G-protein coupled receptors, lack of the first 15 amino acids will not alter the structure and/or function of this receptor. The porcine homolog is subcloned into pBluescript for sequencing.

Based on a high degree of homology to the $GH_4C_1$ receptor, a second somatostatin receptor subtype is cloned. Taking into account conservative substitutions, the brain gene product is ~75% identical at the amino acid level with the pituitary gene product. This particular receptor corresponds to an orphan receptor cloned by Meyerhoff et al., (DNA and Cell Biology 10:689-694, 1991), and is a second somatostatin receptor subtype, probably a brain/gut somatostatin receptor. Using a Xenopus oocyte microinjection system, Moyerhoff et al. could not detect somatostatin receptor function for this particular gene product. It is clear, however, that this particular gene does encode another somatostatin receptor subtype. The brain receptor cDNA is cloned by PCR using 4 oligonucleotide primers. A 5' end antisense strand primer with a unique HindIII site and an internal 3' sense primer are used to amplify the 5' end of the gene. A 3' end antisense strand primer with a unique XbaI site and an internal 5' sense primer are used to amplify the 3' end of the gene. After digestion of each fragment at a unique ApaLI site, the two fragments are ligated together and cloned into pBluescript and pRC/CMV at the HindIII and XbaI sites.

It will be recognized by those skilled in the art that the present invention is not limited to nucleic acid and amino acid sequences comprising the sequences depicted in any of the specific Figures, but also encompasses nucleic acid sequences hybridizing to the disclosed nucleic acid sequences under standard hybridization conditions (e.g., Maniatis et al., Molecular Cloning, 1982 and Sambrook et al., 1989), and proteins encoded thereby, as well as alternate nucleic acid sequences encoding the amino acid sequences of any of the specific figures which, because of the degeneracy of the genetic code, may not hybridize with the depicted nucleic acid sequence.

Plasmids containing a full-length pituitary receptor (pJ2-2) and brain receptor (pJ2-1) have been deposited with the American Type Culture Collection as, respectively, ATCC 68924 and ATCC 68923 on March 10, 1992.

## VII. FUNCTIONAL ANALYSIS OF SOMATOSTATIN RECEPTOR SUBTYPES

The full-length pituitary $GH_4C_1$ and brain somatostatin receptors are cloned into the HindIII and XbaI sites of the expression vector pCR/CMV (Invitrogem). Transfection of Chinese Hamster Ovary (CHO) cells are accomplished using the calcium phosphate procedure.

CHO cells are transfected with the two rat somatostatin receptor subtypes (brain and pituitary). Functional analysis of each subtype is examined by the ability of somatostatin (S-14) to inhibit forskolin-stimulated cyclic AMP accumulation. The diterpene forskolin activates adenylyl cyclase directly by bypassing the normal signal transduction pathway. Cyclic AMP accumulations in intact CHO cells are performed using a kit in accordance with manufacturer's directions (Amersham). CHO wild-type cells are found to be devoid of somatostatin receptor function (Table 5). Forskolin-stimulated cyclic AMP accumulation is not inhibited by S-14 in CHO wild-type cells. In cells that are transfected with either the pituitary or brain cDNAs, forskolin-stimulated cyclic AMP accumulation is inhibited 75% and 63%, respectively, by S-14 (1 $\mu$M). These data indicate clearly that two functional somatostatin receptor cDNAs have been cloned.

## TABLE 5

### Functional Expression of Pituitary and Brain
### Somatostatin Receptor Subtypes in
### Chinese Hamster Ovary Cells:
### Inhibition of Forskolin-Stimulated Cyclic AMP Accumulation

| Receptor Subtype | Basal | Forskolin | Forskolin + S-14 | % Inhibition |
|---|---|---|---|---|
| None (Wild-Type CHO) | 1.7 | 285 | 294 | 0 |
| Brain | 1.3 | 194 | 49 | 75 |
| Pituitary | 4.25 | 131 | 48 | 63 |

Note: Chinese hamster ovary (CHO) cells are transfected with either the brain or pituitary somatostatin receptor subtypes. Inhibition of forskolin-stimulated cyclic AMP accumulation (10 $\mu$M) by somatostatin, S-14 (1 $\mu$M) is compared to wild-type CHO cells. The data are presented as pmoles cyclic AMP/$10^6$ cells.

## VII. PRODUCTION OF ANTIBODIES WITH SOMATOSTATIN RECEPTOR

The technique of hydrophilicity analysis of primary sequence information has been commonly used to identify both hydrophobic potentially membrane-spanning domains and hydrophilic antigenic sites. Analysis of the cloned SRIF receptor by the Hopp and Woods Method (Hopp, T.P., and Woods, K.R., Proc. Natl. Acad., 78:3824, 1981) indicates seven domains rich in hydrophobic residues; this is a common property in the G-protein linked receptor family (Wang, H., Lipfert, L., Malbon, C., and Bahouth, S., J. Biol. Chem., 264:14424, 1989). This model depicts four extracellular regions which are potential targets for binding of anti-receptor antibodies; three extracellular loops (EC-1, EC-2, EC-3) and an N-terminus which contains sites for asparagine-linked glycosylation. For example, the information derived from the pituitary receptor clone is used to make the peptides corresponding to:

EC-1: VHWPFGKAICR
EC-2: RSNQWGRSSCTINWPGESGAWYTG
EC-3: SPTPALKG
N-term: EPYYDMTSNA

Antibodies directed against each of the extracellular regions is analyzed for ligand blocking activity. Since EC-2 is the largest extracellular loop and does not contain N-linked glycosylation sites (carbohydrate may sterically interfere with antibody binding) it may be a preferred target for induction of antibodies which block ligand binding. However, a recent study of antibodies directed against the three EC loops of the thyrotropin receptor indicates a heterogeneity in their biological activities, including the induction of blocking antibodies using the EC-3 Loop as the antigen (Ohmori, M., et al., Biochem. Biophys. Res. Comm., 174399, 1991). Therefore, a broad panel of anti-peptide and anti-receptor antibodies is prepared and carefully evaluated in order to determine the epitopes required for the induction of blocking antibodies.

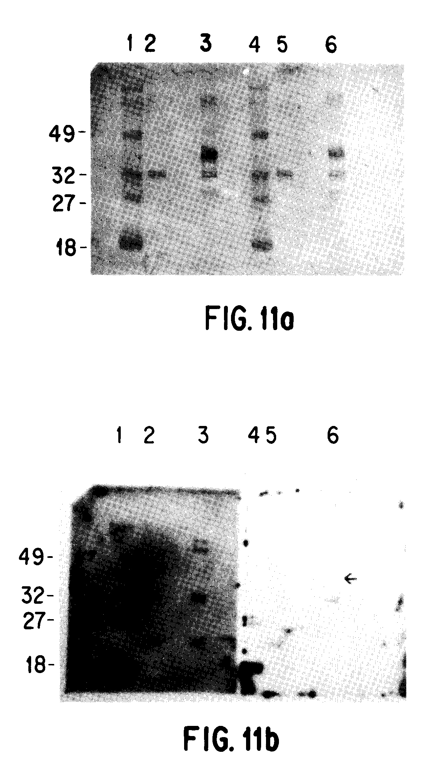

Peptides are produced by conventional solid phase synthesis cleaved by HF, and HPLC purified (van Regenmortal , M.H., In Synthetic Peptides as Antigens, Elsevier, New York, pages 41-93, 1988). Peptides are coupled to a carrier immunogen such as keyhole limpet haemocyanin (KLH) Or ovalbumin via glutaraldehyde by conventional procedures (Coligan, J., et al., In Current Protocols in Immunology, Vol. 1, Wiley-Interscience, New York, 1991) and these conjugates used to immunize mice and rabbits. Animals are given the first immunization of conjugate peptide with Freunds Complete Adjuvant and three weeks later are given weekly booster immunizations with conjugate peptide in Freunds Incomplete Adjuvant. Specific anti-peptide antibodies are detected by their binding to peptide in an ELISA or RIA assay. In those instances where peptides do not adhere to plastic and therefore are not amenable for direct assay then the peptide is conjugated to an unrelated carrier protein which then adheres to the plate. An example of these procedures is in Table 6. A trypsin fragment of the SRIF receptor is amino acid sequenced (LNETTETQR) and is later through DNA sequencing determined to be located near the C-teminus of the receptor. The peptide is synthesized, conjugated to KLH via glutaraldehyde, and used to immunize rabbits. After only seven weeks, the resultant immune sera recognizes both the peptide (Table 6) and the purified receptor from which the original amino acid sequence was obtained (Figure 11).

Table 6 illustrates the radioimmonoassay of rabbit anti-LNETTETQR sera. Microtiter wells are coated with LNETTETQR peptide (2 $\mu$g/well) for 18 hours at 4° C. After washing and a 1-hour blocking step with 2% BSA wells are incubated with rabbit sera for 18 hours at 4° C. Following a 1-hour incubation with $^{125}$I-donkey antirabbit (100,000 cpm/well) the wells are counted in a gamma counter. These data indicate that the rabbit has responded with a anti-LNETTETQR titer of at least 1:2560.

TABLE 6

| Mean cpm of Duplicate Wells | | |
|---|---|---|
| Dilution | Preimmune | Immune |
| 1:160 | 982 | 7490 |
| 1:320 | 983 | 7013 |
| 1:640 | 670 | 5455 |
| 1:1280 | 433 | 5270 |
| 1:2560 | 182 | 3280 |

The specificity of the antibodies is also evaluated by the technique of Western blotting (Towbin, H., et al., Proc. Natl. Acad. Sci., 76:4350, 1979). Specific antibodies recognize an 85 kDa protein in crude $GH_4C_1$ membranes, WGA-eluted glycoproteins, or Streptavidin eluates. After deglycosylation of these samples with Endo-F, the specific antibodies then recognize a 38-kDa protein (Figure 11). Membranes from a cell line devoid of SRIF receptor serves as a negative control (CHO cells). This procedure also gives information about the degree of cross-reactivity of the antibodies with SRIF receptor subtypes from other cell lines ($GH_3$, ATt-20) and tissues (brain, pituitary) and obviates the need for receptor purification from each source. It is beneficial to obtain both tissue-specific antibodies for physiological studios and cross-reactive antibodies for possible use in purification of the receptor from various tissues using immuoaffinity chromatography.

Monoclonal antibodies to the SRIF receptor are prepared using conventional methods (Harlow, H., and Lane, D., In Antibodies: A Laboratory Manual, Cold Spring Harbor Lab, New York, pages 139-240, 1988). The antigens used for immunization include (1) KLH-peptide conjugates corresponding to the extracellular regions as described above, (2) purified receptor from $GH_4C_1$ membranes, (3) purified $GH_4C_1$ receptor deglycosylated with Endo-F and repurified by SDS-PAGE and electroelution, (4) receptor purified from recombinant sources such as transfected CHO cells or baculovirus tested for reactivity to the peptide immunogen by ELISA or protein immunogen by Western blot. Those which display circulating antibodies to the receptor or its peptides are utilized for the preparation of hybridomas. Briefly, spleen cells are removed and fused in the presence of polyethylene glycol to SP2/0 myeloma cells that are deficient in the enzyme hypoxanthine-guanine phosphoribosyl aminopterine-thymidine which selects for true hybrids of both cell types since spleen cells do not grow in culture. Hybridoma supernates are screened for antibody to the receptor by (1) Western blots of purified receptor or WGA-eluted glycoproteins, (2) ELISA using inhibition of radiolabelled ligand binding to membranes. Those hybridomas which are positive are propagated and recloned by limiting dilution or growth in soft agar; this ensures their monoclonal nature. Positive clones are used to induce tumors in mice and accumulate the antibody in ascites fluid.

Both polyclonal and monoclonal IgG antibodies are purified by conventional ammonium sulfate precipitation and Protein A chromatography (Harlow, supra). Antibodies are analyzed for their ability to block radioligand binding to membranes in the standard binding assay described above. Anti-receptor anti-bodies that block ligand binding have boom previously described for insulin (Rogh, R., et al., Proc. Natl. Acad. Sci., 79:7312, 1982), TSH (Marion, S., et al., Endocrinol., 130:967, 1992), and EGF (Sahreiber, A.B., et al., Proc. Natl. Acad. Sci., 78:7535, 1981), among others. Those antibodies that show promise for ligand blocking are tested in vivo using a rat model, e.g., by using indwelling venous catheters on rats in order to monitor GH levels after administraiton of anti-SRIF receptor antibody (Miell, J., et al., J. Endocrinol., 131:75, 1991). Those antibodies which show the most in vivo activity at this point are examined to define their epitopes (if not already determined) on the receptor. Those epitopes represent antigenic fragments of the receptor which when used as immunogens induce antibodies possessing the desired physiological effect of increased levels of GH.

Sequence ID No.:  1

Sequence Type:  Nucleic Acid and Amino Acid

Sequence Length:  167 Amino Acids, 501 Base Pairs

Strandedness:  Single

Topology:  Linear

Original Source Organism:  Rat

Features:  Pituitary Somatostatin Receptor (partial)

```
ATC GAC CGC TAC CTG GCC GTG GTG CAC CCA ATT AAG        36
Ile Asp Arg Tyr Leu Ala Val Val His Pro Ile Lys
  1           5                   10

TCA GCC AAA TGG AGG CGA CCC CGG ACA GCC AAG ATG        72
Ser Ala Lys Trp Arg Arg Pro Arg Thr Ala Lys Met
         15                   20

ATC AAC GTG GCT GTT TGG GGT GTG TCC CTG CTT GTG        108
Ile Asn Val Ala Val Trp Gly Val Ser Leu Leu Val
 25               30                   35

ATT TTG CCC ATC ATG ATA TAC GCT GGC CTC CGG AGC        144
Ile Leu Pro Ile Met Ile Tyr Ala Gly Leu Arg Ser
             40                   45

AAC CAG TGG GGT AGG AGC AGC TGC ACC ATC AAC TGG        180
Asn Gln Trp Gly Arg Ser Ser Cys Thr Ile Asn Trp
     50                   55                   60

CCG GGC GAA TCC GGG GCA TGG TAC ACG GGT TTC ATT        216
Pro Gly Glu Ser Gly Ala Trp Tyr Thr Gly Phe Ile
                 65                   70

ATC TAT GCC TTC ATC CTG GGG TTG CTG GTA CCC CTA        252
Ile Tyr Ala Phe Ile Leu Gly Phe Leu Val Pro Leu
         75                   80

ACC ATC ATC TGT CTC TGC TAC CTG TTC ATC ATC ATC        288
Thr Ile Ile Cys Leu Cys Tyr Leu Phe Ile Ile Ile
 85               90                   95

AAG GTG AAG TCC TCT GGG ATC CGA GTG GGG TCG TCC        324
Lys Val Lys Ser Ser Gly Ile Arg Val Gly Ser Ser
             100                  105
```

21

```
AAG AGG AAA AAG TCA GAG AAA AAG GTG ACC CGA ATG        360
Lys Arg Lys Lys Ser Glu Lys Lys Val Thr Arg Met
    110             115                     120

GTA TCC ATC GTG GTG GCT GTC TTC ATC TTC TGC TGG        396
Val Ser Ile Val Val Ala Val Phe Ile Phe Cys Trp
                125                 130

CTC CCC TTC TAT ATC TTC AAT GTC TCG TCC GTG TCT        432
Leu Pro Phe Tyr Ile Phe Asn Val Ser Ser Val Ser
        135                 140

GTG GCC ATC AGC CCC ACC CCT GCC CTG AAA GGC ATG        468
Val Ala Ile Ser Pro Thr Pro Ala Leu Lys Gly Met
145             150                 155

TTC GAT TTC GTG GTG ATC CTG ACC TAC GCC AAC           501
Phe Asp Phe Val Val Ile Leu Thr Tyr Ala Asn
            160                 165     167
```

Sequence ID No.:  2

Sequence Type:  Nucleic Acid and Amino Acid

Sequence Length:  369 Amino Acids, 1110 Base Pairs

Strandedness:  Single

Topology:  Linear

Original Source Organism:  Rat

Features:  Pituitary Somatostatin Receptor

Properties:

```
ATG GAG ATG AGC TCT GAG CAG TTC AAT GGG AGC CAA         36
Met Glu Met Ser Ser Glu Gln Phe Asn Gly Ser Gln
 1           5                   10

GTG TGG ATA CCT TCT CCC TTT GAC CTC AAC GGC TCA         72
Val Trp Ile Pro Ser Pro Phe Asp Leu Asn Gly Ser
        15                  20

CTG GGG CCA AGC AAT GGC TCC AAC CAG ACA GAG CCA         108
Leu Gly Pro Ser Asn Gly Ser Asn Gln Thr Glu Pro
 25                 30                  35

TAC TAC GAC ATG ACA AGC AAC GCG NTC CTC ACG TTC         144
Tyr Tyr Asp Met Thr Ser Asn Ala Xaa Leu Thr Phe
            40                  45

ATC TAC TTC GTG GTG TGC GTG GTG GGG CTG TGC GGC         180
Ile Tyr Phe Val Val Cys Val Val Gly Leu Cys Gly
        50                  55                  60

AAC ACG CTC GTC ATC TAC GTC ATC CTC CGC TAC GCC         216
Asn Thr Leu Val Ile Tyr Val Ile Leu Arg Tyr Ala
                65                  70

AAG ATG AAA ACC ATC ACC AAC ATT TAC ATC CTC AAC         252
Lys Met Lys Thr Ile Thr Asn Ile Tyr Ile Leu Asn
        75                  80

CTG GCC ATC GCA GAT GAA CTC TTC ATG CTG NGG CTG         288
Leu Ala Ile Ala Asp Glu Leu Phe Met Leu Xaa Leu
 85                 90                  95

CCC TTC TTG GCC ATG CAG GTG GCG CTG GTC CAC TGG         324
Pro Phe Leu Ala Met Gln Val Ala Leu Val His Trp
        100                 105
```

```
CCT TTT GGC AAG GCC ATC TGC CGG GTG GTC ATG ACT        360
Pro Phe Gly Lys Ala Ile Cys Arg Val Val Met Thr
    110             115                 120

GTG GAC GGT ATC AAC CAG TTC ACC AGT ATC TTC TGC        396
Val Asp Gly Ile Asn Gln Phe Thr Ser Ile Phe Cys
                125                 130

TTG ACG GTC ATG AGC ATC GAC CGC TAC CTG GCC GTG        432
Leu Thr Val Met Ser Ile Asp Arg Tyr Leu Ala Val
        135                 140

GTG CAC CCA ATT AAG TCA GCC AAA TGG AGG CGA CCC        468
Val His Pro Ile Lys Ser Ala Lys Trp Arg Arg Pro
145             150                 155

CGG ACA GCC AAG ATG ATC AAC GTG GCT GTT TGG GGT        504
Arg Thr Ala Lys Met Ile Asn Val Ala Val Trp Gly
            160                 165

GTG TCC CTG CTT GTG ATT TTG CCC ATC ATG ATA TAC        540
Val Ser Leu Leu Val Ile Leu Pro Ile Met Ile Tyr
        170                 175                 180

GCT GGC CTC CGG AGC AAC CAG TGG GGT AGG AGC AGC        576
Ala Gly Leu Arg Ser Asn Gln Trp Gly Arg Ser Ser
                185                 190

TGC ACC ATC AAC TGG CCG GGC GAA TCC GGG GCA TGG        612
Cys Thr Ile Asn Trp Pro Gly Glu Ser Gly Ala Trp
        195                 200

TAC ACG GGT TTC ATT ATC TAT GCC TTC ATC CTG GGG        648
Tyr Thr Gly Phe Ile Ile Tyr Ala Phe Ile Leu Gly
205                 210                 215

TTG CTG GTA CCC CTA ACC ATC ATC TGT CTC TGC TAC        684
Phe Leu Val Pro Leu Thr Ile Ile Cys Leu Cys Tyr
            220                 225

CTG TTC ATC ATC ATC AAG GTG AAG TCC TCT GGG ATC        720
Leu Phe Ile Ile Ile Lys Val Lys Ser Ser Gly Ile
    230                 235                 240

CGA GTG GGG TCG TCC AAG AGG AAA AAG TCA GAG AAA        756
Arg Val Gly Ser Ser Lys Arg Lys Lys Ser Glu Lys
                245                 250

AAG GTG ACC CGA ATG GTA TCC ATC GTG GTG GCT GTC        792
Lys Val Thr Arg Met Val Ser Ile Val Val Ala Val
    255                 260
```

```
TTC ATC TTC TGC TGG CTC CCC TTC TAT ATC TTC AAT          828
Phe Ile Phe Cys Trp Leu Pro Phe Tyr Ile Phe Asn
265             270             275

GTC TCG TCC GTG TCT GTG GCC ATC AGC CCC ACC CCT          864
Val Ser Ser Val Ser Val Ala Ile Ser Pro Thr Pro
        280             285

GCC CTG AAA GGC ATG TTC GAT TTC GTG GTG ATC CTG          900
Ala Leu Lys Gly Met Phe Asp Phe Val Val Ile Leu
    290             295             300

ACC TAC GCC AAC AGC TGC GCC AAC CCC ATC CTG TAC          936
Thr Tyr Ala Asn Ser Cys Ala Asn Pro Ile Leu Tyr
            305             310

GCC TTC TTG TCC GAC AAC TTC AAG AAG AGC TTC CAG          972
Ala Phe Leu Ser Asp Asn Phe Lys Lys Ser Phe Gln
        315             320

AAT GTT CTT TGC TTG GTC AAG GTG AGT GGT GCG GAG         1008
Asn Val Leu Cys Leu Val Lys Val Ser Gly Ala Glu
325             330             335

GAT GGG GAG CGG AGC GAC AGT AAG CAG GAC AAA TCC         1044
Asp Gly Glu Arg Ser Asp Ser Lys Gln Asp Lys Ser
        340             345

CGG CTG AAT GAG ACC ACG GAG ACC CAG AGG ACC CTC         1080
Arg Leu Asn Glu Thr Thr Glu Thr Gln Arg Thr Leu
    350             355             360

CTC AAT GGA GAC CTC CAA ACC AGT ATC                     1107
Leu Asn Gly Asp Leu Gln Thr Ser Ile
            365             369

TGA                                                     1110
```

Sequence ID No.:   3

Sequence Type:   Nucleic Acid and Amino Acid

Sequence Length:   141 Amino Acids, 423 Base Pairs

Strandedness:   Single

Topology:   Linear

Original Source Organism:

Features:   Rat

Features:   Brain Somatostatin Receptor

Properties:   5' End of DNA Sequence


ATG TTC CCC AAT GGC ACC GCC CCC TCT CCC ACC TCT     36
Met Phe Pro Asn Gly Thr Ala Pro Ser Pro Thr Ser
 1                5                   10

TCT CCC AGC TCC AGC CCA GGC GGC TGC GGG GAA GGA     72
Ser Pro Ser Ser Ser Pro Gly Gly Cys Gly Glu Gly
            15                  20

GTC TGC AGC AGG GGT CCC GGG TCC GGC GCT GCG GAC     108
Val Cys Ser Arg Gly Pro Gly Ser Gly Ala Ala Asp
 25                  30                  35

GGC ATG GAA GAA CCT GGA CGA AAC TCT TCC CAG AAC     144
Gly Met Glu Glu Pro Gly Arg Asn Ser Ser Gln Asn
                40                  45

GGG ACT TTA AGC GAG GGT CAG GGT AGC GCC ATT CTC     180
Gly Thr Leu Ser Glu Gly Gln Gly Ser Ala Ile Leu
     50                  55                  60

ATC TCT TTC ATC TAC TCC GTG GTA TGC TTG GTG GGA     216
Ile Ser Phe Ile Tyr Ser Val Val Cys Leu Val Gly
                65                  70

CTG TGT GGG AAC TCC ATG GTC ATT TAC GTG ATC CTG     252
Leu Cys Gly Asn Ser Met Val Ile Tyr Val Ile Leu
         75                  80

CGC TAC GCC AAG ATG AAG ACC GCA ACC AAC ATC TAC     288
Arg Tyr Ala Lys Met Lys Thr Ala Thr Asn Ile Tyr
 85                  90                  95

26

```
ATT CTA AAC CTG GCC ATT GCT GAT GAG CTG CTC ATG          324
Ile Leu Asn Leu Ala Ile Ala Asp Glu Leu Leu Met
        100                     105

CTC AGC GTG CCC TTT CTG GTC ACT TCC ACG CTG TTG          360
Leu Ser Val Pro Phe Leu Val Thr Ser Thr Leu Leu
        110                 115                 120

CGC CAC TGG NCC TTT GGC GCG CTA CTT TGC CGC CTG          396
Arg His Trp Xxx Phe Gly Ala Leu Leu Cys Arg Leu
                125                     130

GTG CTC AGC GTG GAT GCA GTC AAN NNN                      423
Val Leu Ser Val Asp Ala Val Xaa Xaa
        135                 140
```

Sequence ID No.:  4

Sequence Type:  Nucleic Acid and Amino Acid

Sequence Length:  132 Amino Acids, 399 Base Pairs

Strandedness:  Single

Topology:  Linear

Original Source Organism:  Rat

Features:  Brain Somatostatin Receptor

Properties:  3′ End of DNA Sequence

```
CAG CAA CGC AAA CGC TCA GAC CGC AAG ATC ACT CTA      36
Gln Gln Arg Lys Arg Ser Asp Arg Lys Ile Thr Leu
 1           5               10

ATG GTG ATG ATG GTG GTG ATG GTT TTT GTC ATC TGC      72
Met Val Met Met Val Val Met Val Phe Val Ile Cys
         15              20

TGG ATG CCT TTC TAC GTG GTA CAG CTA GTC AAC GTG     108
Trp Met Pro Phe Tyr Val Val Gln Leu Val Asn Val
 25          30                  35

TTC GCC GAG CAA GAC GAC GCC ACG GTG AGC CAG TTG     144
Phe Ala Glu Gln Asp Asp Ala Thr Val Ser Gln Leu
             40              45

TCT GTC ATC CTC GGC TAT GCC AAT AGC TGT GCC AAC     180
Ser Val Ile Leu Gly Tyr Ala Asn Ser Cys Ala Asn
     50              55                  60

CCC ATC CTC TAC GGC TTC CTG TCG GAC AAC TTC AAG     216
Pro Ile Leu Tyr Gly Phe Leu Ser Asp Asn Phe Lys
                 65              70

CGC TCT TTC CAG CGC ATC CTG TGC CTC AGC TGG ATG     252
Arg Ser Phe Gln Arg Ile Leu Cys Leu Ser Trp Met
         75                  80

GAT AAC GCT GCG GAG GAG CCT GTT GAC TAC TAC GCC     288
Asp Asn Ala Ala Glu Glu Pro Val Asp Tyr Tyr Ala
85                  90                  95

ACT GCC CTG AAG AGT CGT GCC TAC AGT GTG GAG GAC     324
Thr Ala Leu Lys Ser Arg Ala Tyr Ser Val Glu Asp
             100                 105
```

```
TTC CAG CCT GAG AAT CTG GAA TCT GGA GGC GTT TTC      360
Phe Gln Pro Glu Asn Leu Glu Ser Gly Gly Val Phe
    110                 115                 120

CGT AAT GGC ACC TGC GCT TCC AGG ATC AGC ACG CTT      396
Arg Asn Gly Thr Cys Ala Ser Arg Ile Ser Thr Leu
            125                 130     132

TGA                                                  399
```

## Claims

1. A substantially pure somatostatin receptor, and biologically active fragments thereof.

2. The receptor of Claim 1 which is a pituitary receptor.

3. The receptor of Claim 1 which is a glycoprotein having a molecular weight of about 75-95,000 daltons.

4. The receptor of Claim 3 having a molecular weight of about 80-95,000 daltons.

5. The receptor of Claim 1 which is a nonglycosylated protein having a molecular weight of about 35-40,000 daltons.

6. The receptor of Claim 1 which is purified at least 30,000-fold relative to a membrane-bound somatostatin receptor.

7. The receptor of Claim 1 which is at least about 90% pure.

8. The receptor of Claim 1 comprising the amino acid sequence of Figure 8, 9 or 10, or biologically active fragments thereof.

9. A monoclonal or polyclonal antibody that binds specifically with a somatostatin receptor or fragment thereof.

10. The antibody of Claim 9 which is detectably labelled.

11. An antiidiotypic antibody to a somatostatin receptor.

12. A method for preventing binding of somatostatin to a somatostatin receptor in a host in vivo comprising administering to the host an amount of a substantially pure somatostatin receptor or fragment thereof sufficient to raise antibodies in the host, which antibodies bind specifically to the somatostatin receptor and thereby prevent binding of somatostatin to the receptor.

13. A method for preventing binding of somatostatin to a somatostatin receptor in a host in vivo comprising administering to the host an amount of substantially pure somatostatin receptor or fragment thereof sufficient to bind circulating somatostatin and thereby prevent binding of the somatostatin to the receptor.

14. A pharmaceutical composition comprising an effective amount of a substantially pure somatostatin receptor or biologically active fragment thereof in combination with a pharmaceutically acceptable carrier.

15. A method for imaging in a host a tumor expressing greater than normal amounts of somatostatin receptor comprising administering to the host an effective amount of the monoclonal antibody of Claim 10.

**16.** A method for treating, in a host, a tumor expressing greater than normal amounts of somatostatin receptor comprising administering to the host an effective amount of the antibody of Claim 8 which is conjugated to an antitumor compound, a cytotoxic compound, or a radioisotope.

**17.** An isolated, purified nucleic acid sequence encoding a somatostatin receptor.

**18.** The sequence of Claim 17 which comprises the nucleic acid sequence depicted in Figure 8, 9 or 10 or which hybridizes with a nucleic acid sequence encoding the amino acid sequence depicted in Figures 8, 9 or 10 or biologically active fragments thereof, under standard hybridization conditions.

**19.** The sequence of Claim 17 which encodes the amino acid sequence of Figure 8, 9 or 10, or biologically active fragments thereof.

**20.** A method for recombinantly producing somatostatin receptor comprising culturing a host cell transformed with a nucleic acid sequence encoding a somatostatin receptor, or derivatives of the host cell, under conditions which permit growth of the host cell and expression of the receptor.

**21.** A vector comprising the nucleic acid sequence of Claim 1.

**22.** A host cell comprising the nucleic acid sequence of Claim 1.

**23.** A host cell comprising the vector of Claim 21.

A. S 14 AND BIO − S 14 VS.  [125 − I]TYR 11 − S 14

FIG. 1A

EP 0 508 221 A1

B. S 14, BIO S 28, BIO-C6-S14 AND
   BIO-C12-S14 VS. [125 I]TYR 11-S14

FIG. 1B

FIG. 2

EP 0 508 221 A1

$10^{-8}$ Bio-S28
$10^{-5}$ S14

FIG. 3

$10^{-8}$ Bio S28    +    +
$10^{-5}$ S14    −    +

MW

− 66K

− 45K

− 29K

FIG. 4

MW stds.

(200 ng each)

93

68

45

FIG. 5

FIG.6

FIG.7A

FIG.7B

```
                              27                                          54
ATC GAC CGC TAC CTG GCC GTG GTG CAC CCA ATT AAG TCA GCC AAA TGG AGG CGA
Ile Asp Arg Tyr Leu Ala Val Val His Pro Ile Lys Ser Ala Lys Trp Arg Arg
     ICⅡ
                              81                                          108
CCC CGG ACA GCC AAG ATG ATC AAC GTG GCT GTT TGG GGT GTG TCC CTG CTT GTG
Pro Arg Thr Ala Lys MET Ile Asn Val Ala Val Trp Gly Val Ser Leu Leu Val
                 TMⅣ
                              135                                         162
ATT TTG CCC ATC ATG ATA TAC GCT GGC CTC CGG AGC AAC CAG TGG GGT AGG AGC
Ile Leu Pro Ile MET Ile Tyr Ala Gly Leu Arg Ser Asn Gln Trp Gly Arg Ser
                                              ECⅡ
                              189                                         216
AGC TGC ACC ATC AAC TGG CCG GGC GAA TCC GGG GCA TGG TAC ACG GGT TTC ATT
Ser Cys Thr Ile Asn Trp Pro Gly Glu Ser Gly Ala Trp Tyr Thr Gly Phe Ile

                              243                                         270
ATC TAT GCC TTC ATC CTG GGG TTC CTG GTA CCC CTA ACC ATC ATC TGT CTC TGC
Ile Tyr Ala Phe Ile Leu Gly Phe Leu Val Pro Leu Thr Ile Ile Cys Leu Cys
```

FIG.8A

```
                                     297                                      324
TAC CTG TTC ATC ATC ATC AAG GTG AAG TCC TCT GGG ATC CGA GTG GGG TCG TCC
Tyr Leu Phe Ile Ile Ile Lys Val Lys Ser Ser Gly Ile Arg Val Gly Ser Ser
         TMⅤ
                                     351                                      378
AAG AGG AAA AAG TCA GAG AAA AAG GTG ACC CGA ATG GTA TCC ATC GTG GTG GCT
Lys Arg Lys Lys Ser Glu Lys Lys Val Thr Arg MET Val Ser Ile Val Val Ala
                         ICⅢ                   TMⅥ
                                     405                                      432
GTC TTC ATC TTC TGC TGG CTC CCC TTC TAT ATC TTC AAT GTC TCG TCC GTG TCT
Val Phe Ile Phe Cys Trp Leu Pro Phe Tyr Ile Phe Asn Val Ser Ser Val Ser

                                     459                                      486
GTG GCC ATC AGC CCC ACC CCT GCC CTG AAA GGC ATG TTC GAT TTC GTG GTG ATC
Val Ala Ile Ser Pro Thr Pro Ala Leu Lys Gly MET Phe Asp Phe Val Val Ile
                         ECⅢ                            TMⅦ

CTG ACC TAC GCC AAC
Leu Thr Tyr Ala Asn
```

# FIG.8B

EP 0 508 221 A1

EP 0 508 221 A1

```
                    20                          40                          60
          *          *          *               *          *          *
ATG GAG ATG AGC TCT GAG CAG TTC AAT GGG AGC CAA GTG TGG ATA CCT TCT CCC TTT GAC CTC AAC
Met Glu Met Ser Ser Glu Gln Phe Asn Gly Ser Gln Val Trp Ile Pro Ser Pro Phe Asp Leu Asn

                    80                          100                         120
          *          *          *               *          *          *
GGC TCA CTG GGG CCA AGC AAT GGC TCC AAC CAG ACA GAG CCA TAC TAC GAC ATG ACA AGC AAC GCG
Gly Ser Leu Gly Pro Ser Asn Gly Ser Asn Gln Thr Glu Pro Tyr Tyr Asp Met Thr Ser Asn Ala

                    140                         160                         180
          *          *          *               *          *          *
NTC CTC ACG TTC ATC TAC TTC GTG GTG TGC GTG GTG GGG CTG TGC GGC AAC ACG CTC GTC ATC TAC
Xxx Leu Thr Phe Ile Tyr Phe Val Val Cys Val Val Gly Leu Cys Gly Asn Thr Leu Val Ile Tyr

200                 220                         240                         260
  *          *          *               *          *          *
GTC ATC CTC CGC TAC GCC AAG ATG AAA ACC ATC ACC AAC ATT TAC ATC CTC AAC CTG GCC ATC GCA
Val Ile Leu Arg Tyr Ala Lys Met Lys Thr Ile Thr Asn Ile Tyr Ile Leu Asn Leu Ala Ile Ala

                    280                         300                         320
          *          *          *               *          *          *
GAT GAA CTC TTC ATG CTG NGG CTG CCC TTC TTG GCC ATG CAG GTG GCG CTG GTC CAC TGG CCT TTT
Asp Glu Leu Phe Met Leu Xxx Leu Pro Phe Leu Ala Met Gln Val Ala Leu Val His Trp Pro Phe
```

FIG.9A

EP 0 508 221 A1

```
        340                    360                    380
         *          *          *          *          *          *
GGC AAG GCC ATC TGC CGG GTG GTC ATG ACT GTG GAC GGT ATC AAC CAG TTC ACC AGT ATC TTC TGC
Gly Lys Ala Ile Cys Arg Val Val Met Thr Val Asp Gly Ile Asn Gln Phe Thr Ser Ile Phe Cys


        400                    420                    440                    460
         *          *          *          *          *          *          *
TTG ACG GTC ATG AGC ATC GAC CGC TAC CTG GCC GTG GTG CAC CCA ATT AAG TCA GCC AAA TGG AGG
Leu Thr Val Met Ser Ile Asp Arg Tyr Leu Ala Val Val His Pro Ile Lys Ser Ala Lys Trp Arg


        480                    500                    520
         *          *          *          *          *          *
CGA CCC CGG ACA GCC AAG ATG ATC AAC GTG GCT GTT TGG GGT GTG TCC CTG CTT GTG ATT TTG CCC
Arg Pro Arg Thr Ala Lys Met Ile Asn Val Ala Val Trp Gly Val Ser Leu Leu Val Ile Leu Pro


        540                    560                    580
 *          *          *          *          *          *          *
ATC ATG ATA TAC GCT GGC CTC CGG AGC AAC CAG TGG GGT AGG AGC AGC TGC ACC ATC AAC TGG CCG
Ile Met Ile Tyr Ala Gly Leu Arg Ser Asn Gln Trp Gly Arg Ser Ser Cys Thr Ile Asn Trp Pro


        600                    620                    640                    660
         *          *          *          *          *          *          *
GGC GAA TCC GGG GCA TGG TAC ACG GGT TTC ATT ATC TAT GCC TTC ATC CTG GGG TTC CTG GTA CCC
Gly Glu Ser Gly Ala Trp Tyr Thr Gly Phe Ile Ile Tyr Ala Phe Ile Leu Gly Phe Leu Val Pro
```

FIG.9B

EP 0 508 221 A1

```
              680                        700                        720
     *             *             *             *             *             *
CTA ACC ATC ATC TGT CTC TGC TAC CTG TTC ATC ATC ATC AAG GTG AAG TCC TCT GGG ANC CGA GTG
Leu Thr Ile Ile Cys Leu Cys Tyr Leu Phe Ile Ile Ile Lys Val Lys Ser Ser Gly Xxx Arg Val


              740                        760                        780
     *             *             *             *             *             *
GGG TCG TCC AAG AGG AAA AAG TCA GAG AAA AAG GTG ACC CGA ATG GTA TCC ATC GTG GTG GCT GTC
Gly Ser Ser Lys Arg Lys Lys Ser Glu Lys Lys Val Thr Arg Met Val Ser Ile Val Val Ala Val


              800                        820                        840
     *             *             *             *             *             *
TTC ATC TTC TGC TGG CTC CCC TTC TAT ATC TTC AAT GTC TCG TCC GTG TCT GTG GCC ATC AGC CCC
Phe Ile Phe Cys Trp Leu Pro Phe Tyr Ile Phe Asn Val Ser Ser Val Ser Val Ala Ile Ser Pro


 860                        880                        900                        920
     *             *             *             *             *             *
ACC CCT GCC CTG AAA GGC ATG TTN GAT TTC GTG GTN ATC CTN ACC TAC GCC AAC AGC TGC GCC AAC
Thr Pro Ala Leu Lys Gly Met Phe Asp Phe Val Val Ile Leu Thr Tyr Ala Asn Ser Cys Ala Asn


              940                        960                        980
     *             *             *             *             *             *
CCC ATC CTG TAC GCC TTC TTG TCC GAC AAC TTC AAG AAG AGC TTC CAG AAT GTT CTT TGC TTG GTC
Pro Ile Leu Tyr Ala Phe Leu Ser Asp Asn Phe Lys Lys Ser Phe Gln Asn Val Leu Cys Leu Val
```

## FIG.9C

EP 0 508 221 A1

```
          1000                        1020                        1040
            *              *            *              *            *              *
AAG GTG AGT GGT GCG GAG GAT GGG GAG CGG AGC GAC AGT AAG CAG GAC AAA TCC CGG CTG AAT GAG
Lys Val Ser Gly Ala Glu Asp Gly Glu Arg Ser Asp Ser Lys Gln Asp Lys Ser Arg Leu Asn Glu

       1060                        1080                        1100
         *              *            *              *            *              *
ACC ACG GAG ACC CAG AGG ACC CTC CTC AAT GGA GAC CTC CAA ACC AGT ATC TGA
Thr Thr Glu Thr Gln Arg Thr Leu Leu Asn Gly Asp Leu Gln Thr Ser Ile ***
```

# FIG.9D

```
                         20                          40                          60
           *              *              *              *              *              *
ATG TTC CCC AAT GGC ACC GCC CCC TCT CCC ACC TCT ACT CCC AGC TCC AGC CCA GGC GGC TGC GGG
Met Phe Pro Asn Gly Thr Ala Pro Ser Pro Thr Ser Ser Pro Ser Ser Ser Pro Gly Gly Cys Gly

                         80                         100                         120
           *              *              *              *              *              *
GAA GGA GTC TGC AGC AGG GGT CCC GGG TCC GGC GCT GCG GAC GGC ATG GAA GAA CCT GGA CGA AAC
Glu Gly Val Cys Ser Arg Gly Pro Gly Ser Gly Ala Ala Asp Gly Met Glu Glu Pro Gly Arg Asn

                        140                         160                         180
           *              *              *              *              *              *
TCT TCC CAG AAC GGG ACT TTA AGC GAG GGT CAG GGT AGC GCC ATT CTC ATC TCT TTC ATC TAC TCC
Ser Ser Gln Asn Gly Thr Leu Ser Glu Gly Gln Gly Ser Ala Ile Leu Ile Ser Phe Ile Tyr Ser

  200                     220                         240                         260
           *              *              *              *              *              *
GTG GTA TGC TTG GTG GGA CTG TGT GGG AAC TCC ATG GTC ATT TAC GTG ATC CTG CGC TAC GCC AAG
Val Val Cys Leu Val Gly Leu Cys Gly Asn Ser Met Val Ile Tyr Val Ile Leu Arg Tyr Ala Lys

                        280                         300                         320
           *              *              *              *              *              *
ATG AAG ACC GCA ACC AAC ATC TAC ATT CTA AAC CTG GCC ATT GCT GAT GAG CTG CTC ATG CTC AGC
Met Lys Thr Ala Thr Asn Ile Tyr Ile Leu Asn Leu Ala Ile Ala Asp Glu Leu Leu Met Leu Ser
```

## FIG.10A

EP 0 508 221 A1

EP 0 508 221 A1

```
        340                          360                          380
         *              *             *                 *              *
GTG CCC TTT CTG GTC ACT TCC ACG CTG TTG CGC CAC TGG NCC TTT GGC GCG CTA CTT TGC CGC CTG
Val Pro Phe Leu Val Thr Ser Thr Leu Leu Arg His Trp Xxx Phe Gly Ala Leu Leu Cys Arg Leu

   400                          420     |                 440                          460
    *              *             *      ↓      *                 *              *             *
GTG CTC AGC GTG GAT GCA GTC AAN NNN CAG CAA CGC AAA CGC TCA GAC CGC AAG ATC ACT CTA ATG
Val Leu Ser Val Asp Ala Val Xxx Xxx Gln Gln Arg Lys Arg Ser Asp Arg Lys Ile Thr Leu Met

            480                          500                          520
             *              *             *                 *              *
GTG ATG ATG GTG GTG ATG GTT TTT GTC ATC TGC TGG ATG CCT TTC TAC GTG GTA CAG CTA GTC AAC
Val Met Met Val Val Met Val Phe Val Ile Cys Trp Met Pro Phe Tyr Val Val Gln Leu Val Asn

        540                          560                          580
         *              *             *                 *              *             *
GTG TTC GCC GAG CAA GAC GAC GCC ACG GTG AGC CAG TTG TCT GTC ATC CTC GGC TAT GCC AAT AGC
Val Phe Ala Glu Gln Asp Asp Ala Thr Val Ser Gln Leu Ser Val Ile Leu Gly Tyr Ala Asn Ser

    600                          620                          640                          660
     *              *             *                 *              *             *
TGT GCC AAC CCC ATC CTC TAC GGC TTC CTG TCG GAC AAC TTC AAG CGC TCT TTC CAG CGC ATC CTG
Cys Ala Asn Pro Ile Leu Tyr Gly Phe Leu Ser Asp Asn Phe Lys Arg Ser Phe Gln Arg Ile Leu
```

# FIG.10B

```
            680                          700                          720
             *            *              *             *              *             *
TGC CTC AGC TGG ATG GAT AAC GCT GCG GAG GAG CCT GTT GAC TAC TAC GCC ACT GCC CTG AAG AGT
Cys Leu Ser Trp Met Asp Asn Ala Ala Glu Glu Pro Val Asp Tyr Tyr Ala Thr Ala Leu Lys Ser


            740                          760                          780
             *            *              *             *              *             *
CGT GCC TAC AGT GTG GAG GAC TTC CAG CCT GAG AAT CTG GAA TCT GGA GGC GTT TTC CGT AAT GGC
Arg Ala Tyr Ser Val Glu Asp Phe Gln Pro Glu Asn Leu Glu Ser Gly Gly Val Phe Arg Asn Gly


         800                       820
          *            *            *
ACC TGC GCT TCC AGG ATC AGC ACG CTT TGA
Thr Cys Ala Ser Arg Ile Ser Thr Leu ***
```

# FIG.10C

FIG. 11a

FIG. 11b

European Patent
Office

PARTIAL EUROPEAN SEARCH REPORT
which under Rule 45 of the European Patent Convention
shall be considered, for the purposes of subsequent
proceedings, as the European search report

Application Number

EP   92 10 5164

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | JOURNAL OF BIOLOGICAL CHEMISTRY, vol. 264, no. 31, 5th November 1989, pages 18789-18795, Baltimore, MD, US; F. REYL-DESMARS et al.: "Solubilization and immunopurification of a somatostatin receptor from the human gastric tumoral cell line HGT-1" * Whole document * | 1,3,4,6 -10,12, 14,15 | C 07 K   15/14<br>C 12 P   21/08<br>A 61 K   37/02<br>A 61 K   39/00<br>A 61 K   49/00<br>A 61 K   39/395<br>C 12 N   15/12 |
| X | COMPTES RENDUS DE L'ACADEMIE DES SCIENCES, vol. 308, no. 9, series III, 2nd March 1989, pages 251-254, Paris, FR; F. REYL-DESMARS et al.: "Purification jusqu'à apparente homogénéité du récepteur de la somatostatine de la lignée cellulaire humaine HGT-1 d'origine gastrique" * Whole document *          -/- | 1,3,4,6 -10,12, 14 | |

**TECHNICAL FIELDS SEARCHED (Int. Cl.5)**

C 07 K
C 12 P
A 61 K
C 12 N

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claims 12,13,15 and 16 are directed to a method of treatment of the human/animal body, the search has been carried out and based on the alleged effects of the compound/composition.

| Place of search | Date of completion of the search | Examiner |
|---|---|---|
| THE HAGUE | 08-07-1992 | NOOIJ F.J.M. |

Page    2

European Patent Office

PARTIAL EUROPEAN SEARCH REPORT

Application Number

EP  92 10 5164

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| X | METABOLISM, vol. 39, no. 9, suppl. 2, September 1990, pages 74-77, New York, US; M.J.M. LEWIN et al.: "Molecular characterization of a purified human gastric somatostatin receptor" * Whole document * ----- | 1,3,4,6 -10,12, 14,15 | |
| | | | **TECHNICAL FIELDS SEARCHED (Int. Cl.5)** |

EPO FORM 1503 03.82 (P0410)